# EUROPEAN PATENT APPLICATION

(11) **EP 1 854 410 A1**
(43) Date of publication of application: **14.11.2007**
(21) Application number: 06728573.4
(22) Date of filing: 01.03.2006
(51) Int. Cl.: A61B 5/157, A61B 5/1473, A61B 5/15, A61B 5/151, G01N 33/48, G01N 33/483

(54) **BIOSENSOR COUPLED WITH NEEDLE**

(30) Priority: 02.03.2005 JP 2005057286; 19.04.2005 JP 2005120478
(71) Applicant: National Institute of Advanced Industrial Science and Technology, Tokyo 100-8921 (JP)
(72) Inventor: NAKAMURA, Hideaki, Nat. Inst. Adv. Ind. Science, Tsukuba-shi Ibaraki 3058562 (JP); GOTOH, Masao, Nat. Inst. Adv. Ind. Science Techn., Tsukuba-shi Ibaraki 3058562 (JP); KARUBE, Isao, Nat. Inst. Adv. Ind. Science Techn., Tsukuba-shi Ibaraki 3058562 (JP)
(74) Representative: White, Nina Louise
(86) International application number: PCT/JP2006/303908
(87) International publication number: WO 2006/093206

(57) **Abstract**

There is provided a biosensor unified with a needle in which, since its constitution is simple, its assembling is easy in spite of having strength, collecting of body fluid sample can be conducted surely without waste, a puncturing needle is not polluted by a reagent layer, carrying is easy due to its simple package and hygiene can be maintained even after use. There is also provided a biosensor unified with a needle in which an assembling is easy without deteriorating its strength and collecting a body fluid sample can be surely conducted without waste where a puncturing needle is not polluted with a reagent layer.

A biosensor unified with a needle where a puncturing needle for collecting a body fluid by puncturing the skin of a person to be tested and a main body of a biosensor for conducting the analysis of the body fluid are constituted in a unified manner and the puncturing needle is constituted in such a manner that it penetrates by driving the main body of the biosensor in a vertical direction by an external driving means and punctures the skin. In a biosensor unified with a needle which is constituted in such a manner that a biosensor comprising electrically insulating substrate and cover, a spacer installed in a space sandwiched between them, an electrode and a reagent layer and a puncturing needle for penetrating the skin of a person to be tested and collecting the body fluid are unified, a biosensor unified with a needle which is **characterized in that** a cover part forming an opening for collecting the blood installed at the end of the longitudinal direction of a biosensor has one penetrating hole for a puncturing needle.

## Description

### Technical Field

The present invention relates to a biosensor unified with a needle. More particularly, it relates to a biosensor unified with a needle having a constitution in which a puncturing needle which punctures the skin for obtaining the body fluid (such as blood) and a biosensor which collects and analyzes the body fluid taken out on the surface of the skin are unified.

### Background Art

It has been sometimes carried out already that a diabetic patient himself/herself collects his/her blood and measures the blood sugar level which is a value of glucose in blood. In that case, the patient uses a blood-collecting device called a lancet where a blood-puncturing needle can be attached and detached, collects the blood by puncturing the needle to his/her own finger or arm and transfers the collected blood to a blood sugar level analyzer to measure the blood sugar level. In such a measuring method however, the patient shall have to carry a set of measuring devices comprising sugar level analyzer, lancet, blood-puncturing needle and analyzing element and shall have to carry out the measurement by assembling them upon necessity. Thus, training for its operation needs a long period and considerable time is necessary until a sure measurement can be carried out by the patient himself/herself. Actually, the measurement at the site (such as abdominal wall and earlobe) other than forearm is difficult even by skilful persons. Recently, due to the requirement for providing a lowly invasive sample with less pain, a biosensor which can measure even when a sample amount is 1 µl or less, has been proposed. In the case of such a very small amount, the operation of correctly providing the sample to a biosensor is very difficult. As a result, mistake in the measurement is resulted and there are inconveniences that the patient who is a person to be measured is punctured once again and also that the biosensor is to be exchanged to conduct the measurement once again.
Patent Document 1: Japanese Unexamined Patent Publication No. 09-266898
Patent Document 2: Japanese Examined Patent Publication No. 08-020412

In view of the above, various biosensors unified with a needle have been proposed. Firstly, in the biosensor unified with a needle mentioned in Patent Document 3, each of a puncturing needle and a biosensor is set at different positions in the inner area of a measuring apparatus of a pen type (like a two-colored ballpoint pen) equipped with a driving part for the puncturing needle. After the front end of the pen-like measuring apparatus is attached to the skin of a person to be tested and punctured, the biosensor is exposed to the front end and blood is collected, so that the blood sugar level is measured. In this method however, the troublesomeness that each of needle and biosensor is to be set to measuring apparatus is not solved yet.
Patent Document 3: Japanese Unexamined Patent Publication No. 2000-217804

In a biosensor unified with a needle mentioned in Patent Document 4, a puncturing needle is operated by a driving from outside, and the biosensor unified with a needle has such a unified structure that the puncturing needle moves parallel to the longitudinal direction of a biosensor which is in a long and slender stripe. In this type, since the puncturing needle is exposed from the biosensor, a cover which protects the front end of the puncturing needle is necessary. Further, since the puncturing needle moves in a reagent layer and a conveying path for the collected blood of the biosensor, there is a risk that the surface of the puncturing needle is polluted with the reagent before the puncturing needle punctures the skin of a person to be tested.
Patent Document 4: Japanese Unexamined Patent Publication of national stage of international application No. 2002-056769

Moreover, in the conventional biosensors unified with a needle, the structure is complicated, necessary amount of blood collected as a sample liquid is much. In addition, there is affection of entering the blood into useless spaces. There is also a problem of close contact of an opening for puncturing and collecting the blood to the skin of a person to be tested. There are still more problems such as that high cost is needed for the package for prevention of infection, deterioration of reagent, etc., that consideration in infectious diseases is insufficient in its disposal after use, and that since driving means for puncturing is installed in the measuring apparatus, the biosensor unified with a needle is unable to be resisting to the impact by driving upon puncturing.

### Disclosure of the Invention

### Problems that the Invention is to Solve

An object of the present invention is to provide a biosensor unified with a needle in which, due to its simple construction, assembling is easy without deteriorating the strength, collecting the body fluid sample can be conducted surely without waste, puncturing needle is not polluted by a reagent layer, carrying is easy due to its simple package and hygiene can be maintained even after use.

### Means for Solving the Problems

An object of the present invention as such can be achieved by a biosensor unified with a needle where a puncturing needle for collecting a body fluid by puncturing the skin of a person to be tested and a main body of a biosensor for conducting the analysis of the body fluid are constituted in a unified manner, and the puncturing needle is constituted in such a manner that it penetrates by driving the main body of the biosensor in a vertical direction by a driving means from outside and punctures the skin.

Further, an object of the present invention can be achieved by a biosensor unified with a needle where the main body of a biosensor is equipped with electrically insulating substrate and a cover and a spacer, an electrode and a reagent layer which are placed in a space sandwiched between the substrate and the cover, where the main body of the biosensor is unified with a puncturing needle which punctures the skin of a person to be tested to collect the body fluid, and where a cover part forming an opening for collecting the blood placed a front end in a longitudinal direction of the main body of a biosensor has one penetrating hole for a puncturing needle.

### Advantages of the Invention

In the biosensor unified with a needle in accordance with the present invention, its assembling is easy without deteriorating its strength and its production in a large scale is possible. It also has excellent effects that collecting a body fluid sample can be conducted surely without wastefulness and that a puncturing needle penetrates the site which is different from a reagent layer whereby no contamination by the reagent takes place. There are further characteristics that, due to a simple packing, its carrying is easy and, even after use, it is hygienic whereby it can be used as a cartridge. It is also possible to provide a measuring apparatus for measurement of biosensor unified with a needle being able to be easily carried and dealing with universal plans in which there are available various functions such as a function where data such as temperature of measuring environment and hematocrit value of blood are able to be reflected to calculation of correct blood sugar level (temperature correction and hematocrit value correction), a function for compensation of difference among the lots, a function for automatic illumination of display and puncturing area in a dark place, a function for voice guide and voice recognition dealing with visual disability caused by diabetes mellitus, a function for controlling the measured data due to incorporation of a radio clock, a function for communication of measured data, etc. to medical institutions and a function for charging the battery.

### Brief Description of the Invention

Fig. 1 is a drawing which shows an embodiment of the biosensor according to the present invention.
Fig. 2 is a drawing which shows another embodiment of the biosensor according to the present invention.
Fig. 3 is a drawing which shows still another embodiment of the biosensor according to the present invention.
Fig. 4 is a drawing which shows an embodiment of the biosensor of a suction type according to the present invention.
Fig. 5 is a drawing which shows an embodiment of the biosensor of a suction type having a package form according to the present invention.
Fig. 6 is a drawing which shows another embodiment of the biosensor of a suction type according to the present invention.
Fig. 7 is a drawing which shows still another embodiment of the biosensor according to the present invention.
Fig. 8 is a drawing which shows another embodiment of the biosensor having a package form according to the present invention.
Fig. 9 is a drawing which shows a constitution example of a biosensor unified with a needle according to the present invention.
Fig. 10 is a drawing which shows a constitution example of a biosensor unified with a needle being formed by putting a biosensor in a folder located at the bottom of a housing for a puncturing needle according to the present invention.
Fig. 11 is a drawing which shows a constitution example of a sensor folder of a biosensor unified with a needle having a packing form of the present invention and a using example of the sensor.
Fig. 12 is a drawing which shows a working example of the biosensor unified with a needle according to the present invention.
Fig. 13 is a drawing which shows an example of side and cross section of the biosensor unified with a needle according to the present invention.
Fig. 14 is a drawing which shows another working example of the biosensor unified with a needle according to the present invention by way of its side and cross section.
Fig. 15 is a drawing which shows a constitution example of the biosensor of a suction type unified with needle having the package form of the present invention.
Fig. 16 is a drawing which shows a working example of the biosensor of a suction type unified with needle having the package form of the present invention by way of cross section of its side.
Fig. 17 is a drawing which shows another constitution example of the biosensor of a suction type unified with needle having the package form of the present invention.
Fig. 18 is a drawing which shows another working example of the biosensor of a suction type unified with needle having the package form of the present invention by way of cross section of its side.
Fig. 19 is a drawing which shows still another working example of the biosensor of a suction type unified with needle having the package form of the present invention by way of cross section of its side.
Fig. 20 is a drawing which shows an example of a measuring apparatus installed with a lancet which is to be attached to the biosensor unified with a needle according to the present invention.
Fig. 21 is a drawing which shows an example where the biosensor unified with a needle having a package form according to the present invention is used by attaching to a measuring apparatus installed with a lancet.
Fig. 22 is a drawing which shows another example where the biosensor unified with a needle having a package form according to the present invention is used by attaching to a measuring apparatus installed with a lancet.
Fig. 23 is a drawing which shows still another example where the biosensor unified with a needle having a package form according to the present invention is used by attaching to a measuring apparatus installed with a lancet.
Fig. 24 is a drawing which shows still another example where the biosensor unified with a needle having a package form according to the present invention is used by attaching to a measuring apparatus installed with a lancet.
Fig. 25 is a drawing which shows still another example where the biosensor unified with a needle having a package form according to the present invention is used by attaching to a measuring apparatus installed with a lancet.
Fig. 26 is a drawing which shows an example where measurement is carried out by attaching the biosensor unified with a needle according to the present invention to a measuring apparatus installed with a lancet.
Fig. 27 is a drawing which shows an example of the sensor unified with a needle according to the present invention when the operation upon collecting the blood is conducted is observed from the cross section of the side thereof.
Fig. 28 is a drawing which shows an example of the biosensor of a suction type unified with a needle having a package form according to the present invention when the operation upon collecting the blood is conducted is observed from the cross section of the side thereof.
Fig. 29 is a drawing which shows another example of the biosensor of a suction type unified with a needle having a package form according to the present invention when the operation upon collecting blood is conducted is observed from the cross section of the side thereof.
Fig. 30 is a drawing which shows an example when the biosensor unified with a needle according to the present invention is attached to a measuring apparatus installed with a lancet and measurement is carried out in a dark place.
Fig. 31 is a drawing which shows an example of a container which receives the biosensor unified with a needle having a package form according to the present invention.
Fig. 32 is a drawing which shows an example of a container which receives the biosensor of a suction type unified with a needle having a package form according to the present invention.
Fig. 33 is a drawing which shows a constitution example of the biosensor according to the present invention.
Fig. 34 is a drawing which shows a constitution example of the biosensor equipped with a puncturing membrane according to the present invention.
Fig. 35 is a drawing which shows a constitution example of the biosensor equipped with a non-slip device according to the present invention.
Fig. 36 is a drawing which shows a constitution example of a puncturing part.
Fig. 37 is a drawing which shows an embodiment of a puncturing part.
Fig. 38 is a drawing which shows a constitution example of the biosensor unified with a needle according to the present invention and a using example.
Fig. 39 is a front view of a using example of the biosensor unified with a needle according to the present invention.
Fig. 40 is a side view of a using example of the biosensor unified with a needle according to the present invention.
Fig. 41 is a drawing which shows a constitution example where the biosensor unified with a needle according to the present invention is received in a housing.
Fig. 42 is a drawing which shows a constitution example where the biosensor unified with a needle received in the housing is connected to a measuring apparatus.
Fig. 43 is a drawing which shows an example of a simple package of the biosensor unified with a needle.

### Expression of Reference Letters

- 1: substrate
- 2: cover
- 3: electrically conductive material
- 4: penetrating hole
- 5: puncturing membrane
- 6: non-spacer part
- 7: conveying path for the collected blood
- 8: adhesive layer (spacer)
- 9: terminal
- 10: opening for puncturing and collecting the blood
- 11: air outlet
- 12: penetrating path for a puncturing needle
- 13: electrode reaction part (reagent layer)
- 14: introducing guide for the collected blood
- 15: groove
- 16: tightly closed cap part
- 17: protective film
- 18: strongly adhered part
- 19: detachable part
- 20: non-adhered part
- 21: biosensor unified with a needle (sensor cartridge)
- 22: folder for a puncturing needle
- 23: introducing part for driving from outside
- 24: substrate for fixing a puncturing needle
- 25: guide for coiled spring
- 26: puncturing needle
- 27: coiled spring
- 28: guide for a puncturing needle
- 29: moving part for a puncturing needle
- 30: folder substrate for a puncturing needle
- 31: folder for sensor substrate
- 32: sensor substrate
- 33: folder for sensor cover
- 34: sensor cover
- 35: fixing tool for sensor substrate
- 36: outer side of sensor substrate
- 37: inner side of sensor substrate
- 38: inner side of sensor cover
- 39: outer side of sensor cover
- 40: broken line for perspective observation
- 41: driving from outside
- 42: main body of biosensor
- 43: driving means for a puncturing needle
- 44: introducing part for cartridge of biosensor unified with a needle (folder for cartridge of biosensor unified with a needle)
- 45: trigger part
- 46: operation panel
- 47: display
- 48: operating button
- 49: connector part
- 50: button for initiation of puncturing
- 51: anti-slip device
- 52: cap
- 53: cap equipped with a cramp
- 54: finger
- 55: collecting the blood
- 56: box receiving a sensor unified with a needle
- 57: detaching part for sensor (tightly closing cap and protective film)
- 58: stretchable film
- 59: valve for prevention of backflow
- 60: gas-permeable film
- 61: housing for a puncturing needle
- 62: taper
- 63: hook
- 64: folding line
- 100: cover
- 102: spacer
- 103: substrate
- 104: perforating hole
- 105: adhesive layer
- 106: resist layer
- 107: electrically conductive material
- 108: penetrating path for a puncturing needle
- 109: stopper
- 110: electrode
- 111: terminal
- 112: opening for collecting the blood
- 113: electrode reaction part (reagent layer)
- 114: a puncturing needle part
- 115: coiled spring
- 116: folder for a puncturing needle
- 117: introducing part for driving from outside
- 118: fixing part for coiled spring
- 119: support for a puncturing needle
- 120: puncturing needle
- 121: introducing part for folder of a puncturing needle
- 122: stopper for a puncturing needle folder
- 123: puncturing part
- 124: driving from outside
- 125: main body of biosensor
- 126: folder for a puncturing needle part and biosensor
- 127: sample to be tested
- 128: biosensor unified with a needle
- 129: collecting the blood
- 130: housing for biosensor unified with a needle
- 131: introducing guide for a measuring apparatus
- 132: measuring apparatus
- 133: cap for package
- 134: puncturing membrane
- 135: guide for a puncturing needle
- 136: housing projection for biosensor
- 137: anti-slip device
- 138: upper opening
- 139: lower opening
- 140: introducing part for housing
- 141: connector
- 142: wiring

### Best Mode for Carrying Out the Invention

The biosensor unified with a needle in accordance with the present invention is equipped with a biosensor which can be prepared easily and which can print an electrode pattern and an adhesive layer as a spacer on an insulating substrate such as plastic by a screen printing. The biosensor as such will be illustrated in more detail as follows.

### (Biosensor)

The biosensor includes an insulating substrate, an insulating cover which bonds to the substrate via an adhesive layer acting as a spacer layer, and an electrode on the substrate formed in a space sandwiched between the substrate and the cover. In a space between the substrate and the cover, there is installed a conveying path for the collected blood, which extends from the opening for puncturing and collecting the blood to an air outlet via electrode. A membrane through which a puncturing needle penetrates (hereinafter, it will be referred to as a puncturing membrane), the substrate, the spacer and the cover are layered in this order from the top. A penetrating hole for collecting the body fluid from the lower area is formed in the cover as the opening for puncturing and collecting the blood, a penetrating hole for substrate is also formed at the position different from the penetrating hole of the cover, and the puncturing membrane is installed upon the penetrating hole of the substrate, so that a penetrating path for puncturing needle is formed from the cover to the membrane. The cover is adhered in such manner that an end thereof in the longitudinal direction is not overlapped on the substrate, whereby an opening for collecting the blood is formed. In a cover part forming the opening for collecting the blood, a hole through which a puncturing needle is penetrated is formed. As a result of forming the penetrating hole in such a position, there is achieved an effect that the puncturing needle does not contact the reagent layer formed on the electrode and the needle is not polluted with the reagent. It is also possible that the substrate is installed with an anti-slip device made of rubber at the lower side of an end portion forming the opening for puncturing and collecting the blood which is a part contacting to the person to be tested. As a result, the misalignment between the biosensor unified with a needle and the skin of the person to be tested upon puncturing hardly happens whereby safe and sure puncturing and blood-collecting are now possible. It is also possible that the substrate is installed with an anti-slip device made of rubber at the lower side of an end portion forming the opening for puncturing and collecting the blood which is a part contacting to the person to be tested. As a result, the misalignment between the biosensor unified with a needle and the skin of the person to be tested upon puncturing hardly happens whereby safe and sure puncturing and blood-collecting are now possible.

In the biosensor in which the reagent layer is formed on the electrode where a conveying path for the sample passes, a sample sent from the conveying path for the sample contacts the reagent layer on the electrode whereby the sample reacts with the reagent. This reaction is monitored as an electric change in the electrode.

In the above constitution, surfactant and lipid are also able to be applied around the opening for puncturing and collecting the blood on the cover material and onto the surface of the conveying path for the collected blood. As a result of application of surfactant and lipid, it is now possible to make the transfer of the sample smooth. In the biosensor where the reagent layer is formed on the electrode where the above collected blood is filled, the collected blood sent from the opening for collecting the blood contacts the reagent layer on the electrode whereby the collected blood reacts with the reagent. This reaction is monitored as changes in terms of electricity in the electrode.

In the conveying path for the collected blood near the opening for puncturing and collecting the blood, the pattern of the adhesive layer can be made into a taper structure so as to make the introduction of the collected blood smooth. Similarly, in order to suppress an excessive introduction of the collected blood, it is also possible to make the air outlet narrow or to install a gas-permeable film at the air outlet area.

Further, for an efficient introduction of the collected blood into the biosensor, it is also possible to substitute the material of the opening for puncturing and collecting the blood with a soft material so as to enhance the close adhesion of the opening for puncturing and collecting the blood to the skin of the person to be tested. Gel, elastic material, foamed material, etc. may be used as the soft material. As another efficient introducing method for the collected blood into the biosensor, it is also possible that the surrounding or the contour of the opening for puncturing and collecting the blood is made into a convex structure. In that case, gel, elastic material, foamed material, etc. may be used as a material forming a convex structure. For example, when the contour of the opening for puncturing and collecting the blood is made into a convex structure, the skin of the person to be tested can be raised by pressing the puncturing part against the biosensor unified with a needle before the puncturing whereby the top of the skin can be punctured. The above-mentioned modification of the opening for puncturing and collecting the blood is helpful for a sensuous grasping of the puncturing position by the user as a guide for the opening for puncturing and collecting the blood and is also effective as an anti-slip device.

In order to further grasp the puncturing position visually, an illuminating means from the opening for introduction of the sample may be listed. The illumination is irradiation of light of visible light region and, preferably, laser beam by, for example, a light emitting diode or a light emitting substance such as an organic EL are desirable. When the light emitting substance is a light emitting diode, the puncturing position can be illuminated when plate materials, puncturing membrane, etc. of the puncturing needle housing and biosensor are formed by a transparent material and a light emitting diode is installed in the inner area thereof. Further, when only the plate of the bottom of the puncturing needle housing and the cover of the biosensor among the aforementioned puncturing needle housing and biosensor formed by the transparent material are formed by a color-absorbing material, only the light from the opening for introduction of the sample is irradiated whereby that can be a spotlight for the puncturing position. When an organic EL is used, at least a part of or all of the plate at the bottom of the puncturing needle housing and the cover of the biosensor may be subjected to a surface treatment with an organic EL. As to a supplying source for electricity when the above-mentioned illuminating means is adopted, batteries of the measuring apparatus may be listed. In that case, it is desirable that electricity is supplied through the terminal of the biosensor.

With regard to a plate material for substrate, cover, etc. of the biosensor, plastic, biodegradable material, paper, etc. may be selected so far as they are electrically insulating. A suitable example of the plastic is polyethylene terephthalate.

The electrode may be constituted from carbon, silver, silver/silver chloride, platinum, gold, nickel, copper, palladium, titan, iridium, lead, tin oxide, platinum black, etc. As to the carbon, there may be used carbon nano-tube, carbon micro-coil, carbon nano-horn, fullerene, dendrimer or derivatives thereof. Such an electrode may be made into a plate material by a screen printing method, a vapor deposition method, a sputtering method, a foil-adhering method, a plating method, etc.

The electrode may be a two-electrode system formed from working electrode and counter electrode, a three-electrode system formed from working electrode, counter electrode and reference electrode or an electrode system comprising more electrodes. When a three-electrode system is adopted, measurement of transfer speed of the collected blood introduced into a conveying path can be conducted besides the electrochemical measurement of the substance to be measured whereby a hematocrit value can be measured. It is also possible to be constituted from two or more sets of electrode systems.

An adhesive layer can also be formed by a screen printing method. As to an adhesive, that of an acrylate resin type may be used for example and the adhesive layer formed in a thickness of about 5 to 500 mm or, preferably, about 10 to 100 mm acts as a spacer as well. Reagent may also be contained in the adhesive layer.

Further, in the biosensor, the electrode may be regulated by a resist layer and the resist layer can be easily formed by a screen printing or the like as well.

A reagent layer is formed by a screen printing method or a dispenser method and fixation of the reagent layer to the electrode surface or plate material surface can be carried out by an adsorption method accompanied by drying or by a covalent bond method.

When the biosensor unified with a needle in accordance with the present invention is constituted for the measurement of blood sugar level, a reaction reagent containing, for example, a glucose oxidase which is an oxidizing enzyme and potassium ferricyanide as a mediator may be adopted as the reagent arranged at the reaction part of the biosensor.

When the above-mentioned reaction part is dissolved in the blood, an enzyme reaction starts and, as a result, potassium ferricyanide coexisting in the reaction layer is reduced and potassium ferricyanide which is an electron carrier of a reduced type is accumulated. Its amount is proportional to the substrate concentration or, in other words, to the glucose concentration in blood. The electron carrier of a reduced type accumulated for certain time is oxidized by an electrochemical reaction. An electron circuit in the main body of the measuring apparatus calculates and determines the glucose concentration (blood sugar level) from anode current measured at that time and, as mentioned already, it is displayed on the display located on the surface of the main body.

The biosensor apparatus unified with a needle in accordance with the present invention is equipped with any of the above-mentioned biosensor, a measuring part which measures the electrical value of the biosensor at the electrode and a display part where the measured value at the measuring part is displayed. As to a measuring method at this measuring part, a potential step chronoamperometry method, a coulometry method, a cyclic voltammetry, etc. are used. It is further possible that the present apparatus is installed with electric wave such as Bluetooth (registered trade mark) as a wireless means.

### (Biosensor unified with a needle)

In a biosensor unified with a needle, a puncturing needle, which penetrates the biosensor at the site being different from the reagent layer and collects a body fluid from the skin of a person to be tested, is received in the same container together with the biosensor in such a manner that the puncturing needle and the biosensor are separated into upper and lower parts of the container with a substrate as a boundary, whereby a unified formation is resulted.

In a puncturing needle housing in a biosensor unified with a needle, an elastic body by which a puncturing needle is projected outside by driving from outside and then immediately returns to the original position and a guide by which a puncturing needle is driven according to the regulation are installed. Since the biosensor unified with a needle can be in a simple package, it is always hygienic before and after use. Further, since it is also in a cartridge type, it can be received in a container exclusively therefor which is convenient for carrying whereby anyone can attach it to a measuring apparatus easily and surely. As a result of adoption of the mode of a cartridge type as such, convenience of the biosensor unified with a needle is now able to be significantly improved as compared with the conventional method. Now the structure in the puncturing needle housing will be mentioned in more detail.

A puncturing needle is received in a housing which is to drive in the vertical direction. It is necessary that, in the puncturing needle housing, there is equipped a mechanism by which the puncturing needle driven by the driving means from outside penetrates the biosensor and, after puncturing the skin of a person to be tested, it returns to the original position. An example of a method satisfying such a condition is that, in addition to the housing for a puncturing needle, a guide which makes the deviation from the orbit minimum may be installed in the inner area as well. In the guide, the surrounding of a puncturing needle installed below the fixing substrate for a puncturing needle being driven from outside for example is protected by a cylindrical guide. Similarly, a cylindrical guide having less radius than the above guide is installed on the folder substrate for a puncturing needle in the puncturing needle housing whereby, upon puncturing, a guide on the folder substrate for a puncturing needle comes into the inner area of the guide of a puncturing needle fixing substrate with a small gap. As a result, the orbit of the puncturing needle can be ensured as regulated. With regard to the puncturing needle for collecting the body fluid from the skin of a person to be tested, it is necessary in some cases that the needle penetrates the puncturing membrane which will be mentioned later and further punctures the person to be tested. Therefore, it has a strength endurable against that and is sharp and, in order to suppress the pain upon puncturing, a fine puncturing needle is preferred. To be more specific, a commercially available product such as that of 21 to 33 gauges manufactured by Thermo may be used. The puncturing needle may be either a hollow needle or a needle without hollow so far as it can break through the skin of a person to be tested.

An elastic body which returns the puncturing needle projected to outside by the external driving to the original position is also necessary. With regard to such an elastic body, there will be a coiled spring for example. The coiled spring can be installed at the outside of a cylindrical guide formed beneath the fixing substrate for puncturing needle in a housing for a puncturing needle. In that case, due to the presence of a guide regulating the orbit of the puncturing needle, it is possible to regulate the movement of the coiled spring in the up-and-down direction only. Besides the coiled spring, a stretchable film may be used as well. In that case, when the stretchable film is fixed to both of a substrate for fixing a puncturing needle and an upper area of a housing for a puncturing needle housing, the same effect as a coiled spring can be achieved. Thus, when a substrate for fixing a puncturing needle moves from up to down by driving from outside, the distance between the fixing substrate for a puncturing needle and the upper area of a housing for a puncturing needle becomes big whereby the stretchable film is extended. A reaction thereof happens by elimination of the driving from outside and the puncturing needle can return to the original position.

The puncturing needle forms puncturing part together with an elastic body which returns it immediately to the original position when it projects outside by the driving from outside, and a puncturing needle folder which drives the puncturing needle as regulated. The puncturing part is received in the housing together with the biosensor so that it is further driven in a vertical direction with respect to the skin. In the housing, it is necessary to install a mechanism by which the puncturing needle driven by a driving means penetrates the cover forming the opening for collecting the blood of the biosensor and, after puncturing the skin of a person to be tested, it return to the original position. As an example for satisfying such a condition, it is also possible to install a puncturing needle part - biosensor folder regulating the steric arrangements of the puncturing needle and the biosensor. When the puncturing needle part - biosensor folder is supported by such a manner that, for example, an area near the center of the reaction part of the puncturing part receiving the driving from outside is supported by a cylindrical guide (a puncturing needle guide) whereby it is possible that, upon puncturing, the puncturing needle moves in the puncturing needle guide in an orbit as regulated. It is also preferred that, in order to conduct a puncturing to the skin of a person to be tested, the front end forming an opening for collecting the blood in the biosensor is slightly projected from the housing so that there is provided a structure where a user can sensually grasp the puncturing position easily.

The puncturing needle penetrates a penetrating hole formed on the cover for the biosensor and, in view of prevention of flowing out of the blood from the penetrating hole, the angle between the puncturing needle and the biosensor upon penetration is fixed in 20 to 90° or, preferably, 30 to 60° by a puncturing needle and biosensor folder.

With regard to an elastic body installed in a puncturing part which returns the puncturing needle projected to outside the biosensor by the driving from outside to the original position, a coiled spring may be exemplified. The coiled spring encloses the outer surrounding of a support of the puncturing needle and is fixed, for example, between an introducing part for driving means for puncturing from outside formed on the upper area of a puncturing needle support and a puncturing needle folder for regulating the orbit of the puncturing needle. When the puncturing needle folder is received in a puncturing needle part - biosensor folder, the whole puncturing needle part including the coiled spring can be inserted into the puncturing needle part - biosensor folder whereby a simple assembling is possible.

The penetration hole formed in the cover is preferably equipped with a puncturing membrane or a shutter which opens and closes linking with the movement of a puncturing needle or, more preferably, with a puncturing membrane. The puncturing membrane is formed in an outer or inner area of the cover so as to clog the penetration hole of the biosensor or, preferably, in an inner area thereof from a viewpoint that the amount of the collected blood is made low and introduction of the collected blood to the inner area of a sensor due to capillary phenomenon after the puncturing is not disturbed. The membrane where the thickness is not more than 100 µm or, preferably, 10 to 50 µm is used in such a view that it makes the passage of the puncturing needle better.

When a stretch film is used for the above-mentioned object, airtightness of the inner part of the puncturing needle housing can be easily achieved. Accordingly, when a mechanism which adjusts the inner pressure is installed in the housing for a puncturing needle, it is now possible to intake the body fluid after being punctured into the inner part of a biosensor by suction. As a tool for achieving such a mechanism, a valve for prevention of backflow may be exemplified. Thus, when the driving from outside works, the inner part of the housing for a puncturing needle temporarily becomes a high pressure. After the pressure is lowered by a valve for prevention of the backflow, the stretchable film tends to return to the original state. As a result, the pressure in the housing becomes low as compared with the outer pressure and that can work as a sucking force for collecting the body fluid. Further, when the stretching property of this stretchable film is changed, sucking force for collecting the blood can be also changed and, therefore, it is also possible to reflect the difference in the physical condition (viscosity of blood) of a user.

Now, a puncturing membrane will be illustrated. It is necessary that the puncturing membrane located at the substrate of a biosensor does not disturb the introduction of the collected blood into the inner part of a sensor by capillary phenomenon after puncturing. In addition, when the puncturing membrane is a material which does not change the volume in a conveying path accompanied by the introduction of the collected blood, it is possible that the plate material of the substrate is eliminated and the puncturing membrane is endowed with a role of a substrate. In that case, when the puncturing membrane closely contacts the puncturing needle folder substrate, the puncturing membrane is not necessary to be a hard material and, therefore, a soft material through which a puncturing needle is apt to penetrate can be selected. A puncturing membrane satisfying such a requirement is preferred to be formed by natural material, plastic, biodegradable material, shape memory alloy, etc. and, more preferably, the natural material is paper, collagen, cellulose, cotton, etc., the plastic is a foamed material or, particularly, artificial skin, elastic material, film-forming material, etc. and the film-forming material is cellophane, polyvinylidene chloride, polyvinyl chloride, polyvinyl acetate, polyimide, polyethylene, polyethylene terephthalate, polyvinyl alcohol, polylactic acid, polyester, polyamide, ethylene-vinyl alcohol copolymer, fluorine resin, etc.

In addition, as to the structure of the puncturing membrane, it may have a multi-layered structure with two or more layers where materials are different. For example, in the case of a two-layered membrane, the lower membrane may be a complete film without holes while the upper one may be a bulky film for filling up a hole formed by puncturing. The puncturing membrane is preferred to be a thin membrane having a thickness of not more than 100 µm in view of making the passage for a puncturing needle better. It is also possible that, instead of a puncturing membrane, a shutter which opens and closes linking with the movement of the puncturing needle is installed at a cover part of a biosensor.

When a puncturing membrane is arranged independently of the reagent layer, adhesion of the reagent to the point of a needle by puncturing can be prevented whereby that is safe and hygienic.

With regard to a puncturing needle used for a biosensor unified with a needle, it is necessary to penetrate the puncturing membrane and further puncturing a person to be tested and, therefore, it is desired to have certain strength and to be sharp. In order to suppress the pain upon puncturing, a fine puncturing needle is preferred. Further, since a puncturing needle is to be hygienically received in the housing until being used, a photocatalystic function which is antibacterially and antivirally effective may be given onto the surface of the needle. In that case, a membrane of titanium oxide or titanium dioxide is preferred.

As to a method for a hygienic storage of the biosensor unified with a needle according to the present invention until being used, a simple packing method using an adhesive protective film (hereinafter, it will be referred to as a protective film) or the like is available. In a biosensor unified with a needle, packing for an opening for collecting and puncturing the blood and/or an air outlet contacting to the outer air and packing for the upper area of a housing for a puncturing needle are needed. The above-mentioned packing means are also able to prevent the exposure of a puncturing needle and, therefore, it is important not only in hygiene but also in safety.

In the packing of a biosensor of the present invention, packing for an opening for puncturing and collecting the blood which directly contacts the outer air is effective. A simple packing material which is most appropriate to be used therefor is a protective film which can be adhered and detached. When at least a non-adhesive layer is installed in the protective film in addition to an adhesive layer which can be adhered and detached, that area can be used as a picking-up part for conducting adherence and detachment of the protective film. Further, when at least a part of the protective film is fixed to a biosensor substrate using a strong adhesive layer, a re-packing by the protective film after detachment is possible.

In the above-mentioned biosensor, there is a cutting line on a boundary between a part containing no electrode of insulating substrate and a part containing an electrode of insulating substrate and the cutting line is also formed on a cover material corresponding to the cutting line on the substrate whereby, when a part containing no electrode of insulating substrate is cut out along the cutting line, a biosensor where an air outlet is opened. In that case, it can adopt such a structure that at least a part of a cap part for an opening has a part which strongly adheres to a protective film and further that the protective film is equipped with a detachable adhesive part at the part adhering to the main body of the biosensor whereby, when the cap is detached from the main body of a biosensor in using the biosensor, the protective film can also be detached. According to such a structure, it is possible that a packed thing comprising cap and protective film can be re-adhered to the original state after using the biosensor.

Similarly, when the packing form of the biosensor unified with a needle is taken into consideration, the terminal of the biosensor is to be protected as well. In that case, the terminal on the insulating substrate can be coated by a cover. In the cover for coating the terminal, it is also possible that no adhesive layer is formed but a folding-back line which can fold back is formed and, along the folding-back line, an external terminal may be exposed. As to the folding-back line in that case, a perforation and a rift may be exemplified.

As to a simple packing method for upper and lower areas of a housing when a biosensor is received in the housing, the use of a cap may be firstly listed. When a cap is merely inserted into an upper area of a housing, etc., the simplest packing is possible. When a cap is connected to a cramp of the housing, then, during the biosensor is used, the cap is kept open while, after use, the cap is applied again to cover whereby the biosensor unified with a needle can be disposed without affection by an infectious disease, etc. As such, when a cap is used, there is an advantage that the surroundings of the introducing part for external puncturing and driving of the housing and the opening for collecting the blood located at the lower part are able to be packed separately.

As to the second means, the use of a protective film will be exemplified. The film can be adhered and detached the same as that used for a biosensor and a non-adhesive part may be formed while at least a part of the protective film may be fixed to a housing for a puncturing needle by a strong adhesive layer. As a result of installing a strong adhesive layer, the protective film maintains a detached state during the use of the biosensor unified with a needle while, after use, re-packing is conducted whereby the biosensor unified with a needle after use can be disposed without affection by infectious disease, etc.

Further, as the third means, the case where the above-illustrated stretchable film is formed on the upper part of the housing for a puncturing needle can be directly applied. Thus, in the case of this embodiment, inner area of the housing for a puncturing needle is kept airtightness whereby the biosensor cartridge unified with a needle including the housing for a puncturing needle is made into a state where no packing is necessary. In such an embodiment, no simple packing of the housing part for puncturing needle is necessary and, therefore, both operation (labor) for unpacking before use and operation for re-packing are able to be eliminated and that is the most convenient packing means among the examples mentioned hereinabove.

Together with a measuring apparatus, as a means for improving a portability of the cartridge for biosensor unified with a needle, it is also possible to use a box in which plural cartridges are able to be received together. When the receiving box is, for example, in such a type where even ten cartridges are able to be received, its size may be made in a size of a ball-point pen. The reason why the cartridge of the present invention can be carried in such a form is that things including elements which are usually difficult for packing such as terminal for a biosensor have now a shape which is safe and users are hardly injured.

### (Measuring apparatus for biosensor unified with a needle)

An example of the measuring apparatus for the biosensor unified with a needle according to the present invention is characterized in that operability and durability by which measurement using the biosensor unified with a needle in a cartridge type can be conducted repeatedly and surely and that its size and shape as a ballpoint pen make its carrying easier. For example, a hook for hanging it in a breast pocket or in an inside pocket of suits may be installed on the upper part of the measuring apparatus.

Further, this measuring apparatus is in nearly the same size as a commercially available lancet and is equipped with a mechanism for generating a power for driving the common puncturing needle. As to a measuring apparatus, that which ensures operability and durability by which the measurement using a biosensor unified with a needle of a cartridge type can be repeatedly and surely carried out and is easy for its carrying is used. A measuring apparatus having the following mechanism is used. Thus, a biosensor unified with a needle of a cartridge type is slid transversely to an introducing part located below and terminal of the biosensor connected to a connector of the measuring apparatus whereby the state where the measurement is possible is resulted, then a trigger is pulled for giving a puncturing drive into the cartridge whereby preparation for the measurement is finished and the a switch of the puncturing initiation button is pushed so that the puncturing, collecting the blood and measurement are automatically carried out whereupon the measured result is finally achieved.

An example of the characteristics in the structure of the measuring apparatus will be mentioned in more detail. Thus, in this measuring apparatus, a driving part for a puncturing needle and a measuring apparatus part are unified and the driving part for a puncturing needle is constituted from a trigger part, a puncturing initiation button part and a driving part by an elastic substance such as spring. On the other hand, in the measuring apparatus part, a fundamental constitution comprises introducing part for the cartridge, connector, circuit for electrochemical measurement, memory part, operation panel, measuring part where electrical value of the biosensor at electrode is measured and display part where measured value at the measuring part is displayed and it is also possible to install electric wave as a wireless means such as Bluetooth (registered trade mark). As a result of such a slide structure, a puncturing driving is received at the state where the cartridge is surely held and, therefore, strength of the measuring apparatus as a whole can be enhanced.

As a result of the above-mentioned slide structure, a puncturing driving is received at the state where the cartridge is surely held and, therefore, strength of the measuring apparatus as a whole can be enhanced.

With regard to the driving for puncturing of the measuring apparatus, a mechanism where the upper part of the cartridge is stricken in a vertical direction and then quickly returns is recommended and it is further preferred to have a mechanism where the depth of puncturing the skin of a person to be tested can be adjusted. In order to ensure the puncturing to the skin of a person to be tested, it is also possible that an anti-slip device is attached to the bottom of the measuring apparatus such as to the area near the bottom of a connector which is a contact point to the cartridge. In that case, an anti-slip device made of rubber is preferred. Similarly, with regard to a puncturing initiation button, modification by such an anti-slip device may be conducted as well. In addition, there may be also equipped with a function in which the above-mentioned illumination function automatically works linking with pulling of the trigger for driving the puncturing of the measuring apparatus and the light irradiated from the part wherefrom the collected blood is introduced illuminates the puncturing part of a person to be tested. As a result, a series of operations of puncturing, collecting the blood and measurement including visually handicapped persons are able to be more surely carried out.

As to another illuminating means in the measuring apparatus of the present invention, there may be equipped with a mechanism in which a light-detecting element such as a photodiode incorporated in the measuring apparatus automatically measures the illuminance and illuminates, if necessary, the puncturing part and the display part. As a result, even in a somewhat dark environment such as a dark place or toilet, measurement can be conducted without turning the switch of the illumination on. Further, when the mechanism works only when a specific operation is done such as upon calling the data or upon operation of the display part, convenience of the measuring apparatus of the present invention is further improved. However, when such a convenient mechanism is adopted, there is a possibility that consumption of electricity increases. As a countermeasure therefor, the measuring apparatus may be made into such a system where charge is possible and, further, charging by solar batteries may be equipped therewith. It is also possible that auxiliary batteries are installed inside.

With regard to a measuring method of the measuring apparatus at the measuring part, although there is no particular limitation, a potential step chronoamperometry method, a coulometry method or a cyclic voltammetry method may be used.

It is also possible to be equipped with various correction mechanisms. As to an important correction in the measurement of blood sugar, there are temperature correction and hematocrit value. Since activity of an enzyme used for the measurement of blood sugar level is apt to be affected by temperature, there are many cases where temperature correction is important especially when measuring time is made short. Hematocrit value is also a factor for affecting the measurement of blood sugar level. Measurement of hematocrit value can also be presumed, for example, from the moving speed of collecting the blood in a biosensor. As to a means therefor, it is also possible that, for example, three electrodes are used, the time when the collected blood passes the first two and the time when it passes the third one are measured and the moving speed of the collected blood is calculated from the time difference and distance between the electrodes.

Besides the above, it is necessary to suppress the variations in measured values depending upon the difference in lots, etc. of a biosensor. In that case, a function by which correction is automatically done for each lot may be equipped therewith.

In order to control the data of blood sugar level correctly determined by the above-mentioned means, etc., it is also necessary to control the correct time. In that case, elements of a radio clock which has been able to be available at a low cost recently may be incorporated into a measuring apparatus. As a result, it is now possible that the data in the past are memorized under a correct time control.

As another means for controlling the measured data, transfer of the data to other medium may be listed. Examples thereof are a method where data are sent from a measuring apparatus to PC or the like via a cable for data transmission and a use of communication means such as by electric wave. When such a communication means is applied to a measuring apparatus, troublesomeness such as connection of cord can be solved.

Further, when voice guidance and voice recognition function are installed in a measuring apparatus, many users including those having physical disability are able to recognize its easiness in handling. For example, with regard to voice guidance, operation proceeding, related precautions, etc. are able to be guided to a user on a real-time basis when the measuring apparatus is actually used to measure the blood sugar level whereby frequency of mistakes in the operation can be made low. Furthermore, report of the measured result, data in the past, etc. are able to be conducted by calling by voice.

In addition, the voice recognition function also results in improvement in operability. For example, it is now possible that calling of the data in the past, illumination of the puncturing area, etc. are conducted by merely speaking to a measuring apparatus without conducting operation buttons. Moreover, depending upon the state how the measuring apparatus is set, i.e., when the apparatus is set longitudinally or transversely, it is preferred that a display is automatically switched in a longitudinal or transverse position depending upon the above. Alternatively, it is preferred to be equipped with a function where a display is continuously changed depending upon the angle how a measuring apparatus is set whereby letters, etc. on the screen appears in parallel. As a result of such a function, it is no longer necessary that a user turns his/her eyes depending upon how the letters appear on the display.

As such, the biosensor unified with a needle, cartridge and a measuring apparatus for the biosensor unified with a needle in accordance with the present invention are able to meet with the universal plan where there is no limitation for the users.

Now the biosensor unified with a needle, the cartridge and the measuring apparatus for a biosensor unified with a needle in accordance with the present invention will be illustrated in detail by referring to drawings for each of them although the present invention is not limited to the following Examples so far as it is out of the gist of the invention.

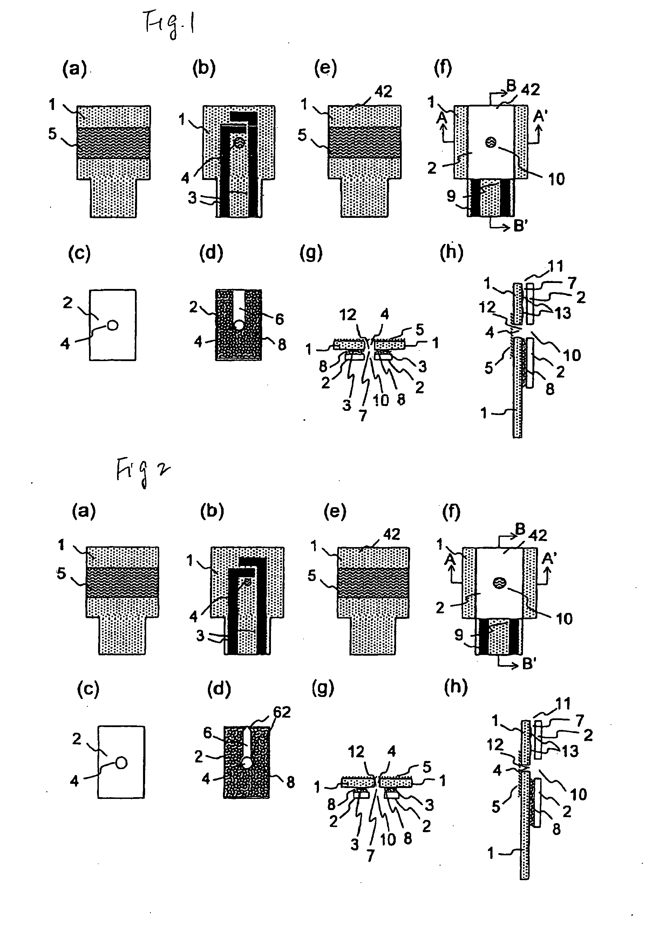

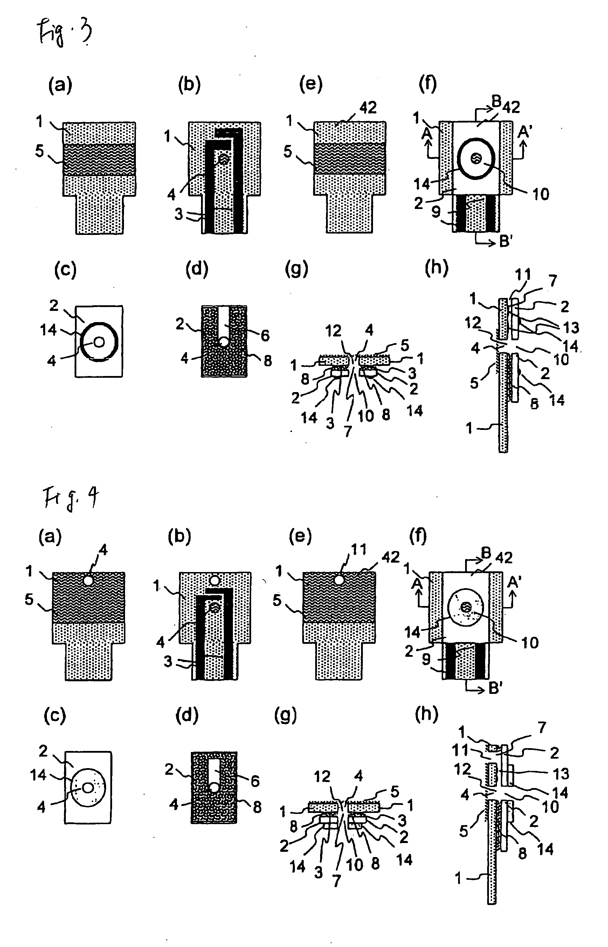

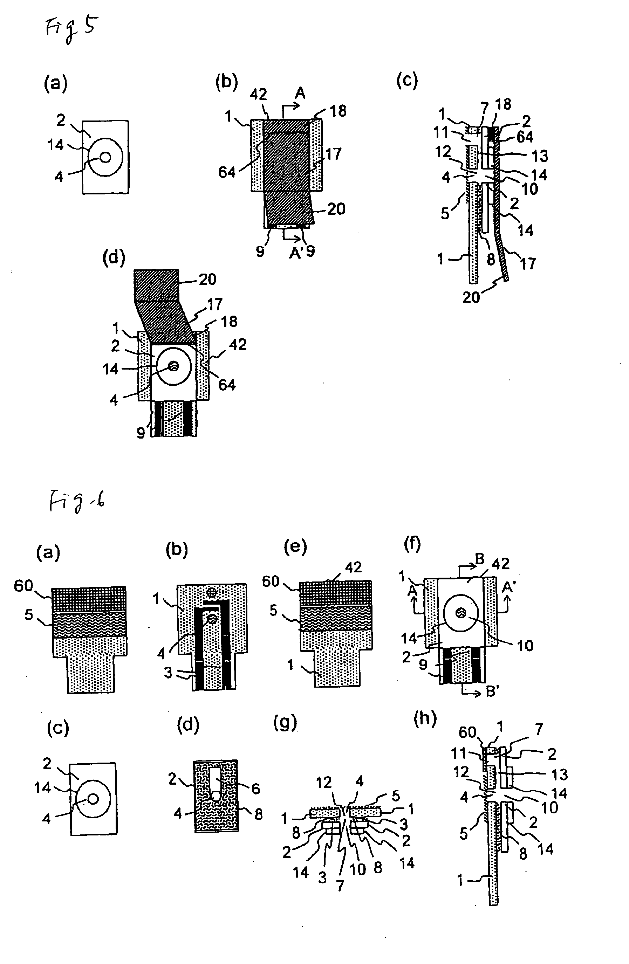

Fig. 1 shows an example of a constitutional drawing of a biosensor of the present invention. Fig. 1(a) shows the outer surface of a substrate 1, and a puncturing membrane 5 is adhered on the substrate 1. Fig. 1 (b) shows the inner surface of the substrate 1, and two electrically conductive materials 3, 3 are arranged on the substrate 1, and a penetrating hole 4 is at the center in the widthwise direction of the substrate. Fig. 1(c) shows the outer surface of a cover 2, and a penetrating hole is formed at the center of the cover 2. Fig. 1(d) shows the inner surface of the cover 2, and an adhesive layer 8 is formed on the cover 2 for a spacer while, in the area where no adhesive layer 8 is formed, a penetrating hole 4 and a non-spacer part 6 are formed. Fig. 1 (e) shows a side of the substrate 1 of the biosensor 42 after being assembled. Fig. 1(f) shows a side of the cover 2 of the biosensor 42 after being assembled. Here, the penetrating hole 4 shown in Fig. 1(c) is an opening 10 for puncturing and collecting the blood, and two electrically conductive materials 9, 9 uncovered with the cover 2 are terminals. Right and left substrates 1 protruded from the cover 2 hang a sensor cover folder 33 (not shown) of the bottom of a housing 61 for the puncturing needle which will be shown later and is formed for prevention of detachment of the main body of the biosensor 42 from the housing 61 for the puncturing needle due to the shock upon puncturing. Fig. 1(g) is a cross section along A-A' in (f). On the upper side of the substrate 1, a puncturing membrane 5 is formed and an electrically conductive material 3, an adhesive layer 8 as a spacer and a cover 2 are divided by a penetrating path 12 for a puncturing needle downward from the substrate 1. In Fig. (g), a puncturing needle penetrates from the upper side of a puncturing membrane 5 through a puncturing membrane 5, passes a penetrating path 12 for a puncturing needle, projects the opening for puncturing and collecting the blood and returns to the original position. During such a process, the penetrating hole for a puncturing membrane 5 which was once opened is closed or become small whereby the collected blood flowing from the opening 10 for puncturing and collecting the blood can introduce to an electrode reacting part 13 on a conveying path 7 for the collected blood without disturbing a capillary phenomenon. Similarly, Fig. 1(h) shows a cross section along B-B'. The collected blood obtained by penetration of the puncturing needle through the puncturing membrane 5 is taken from the opening for puncturing and collecting the blood and sent to an electrode reaction part 13. In the biosensor of Fig. 1, an air outlet 11 is formed on the upper part of the biosensor as shown in (h).

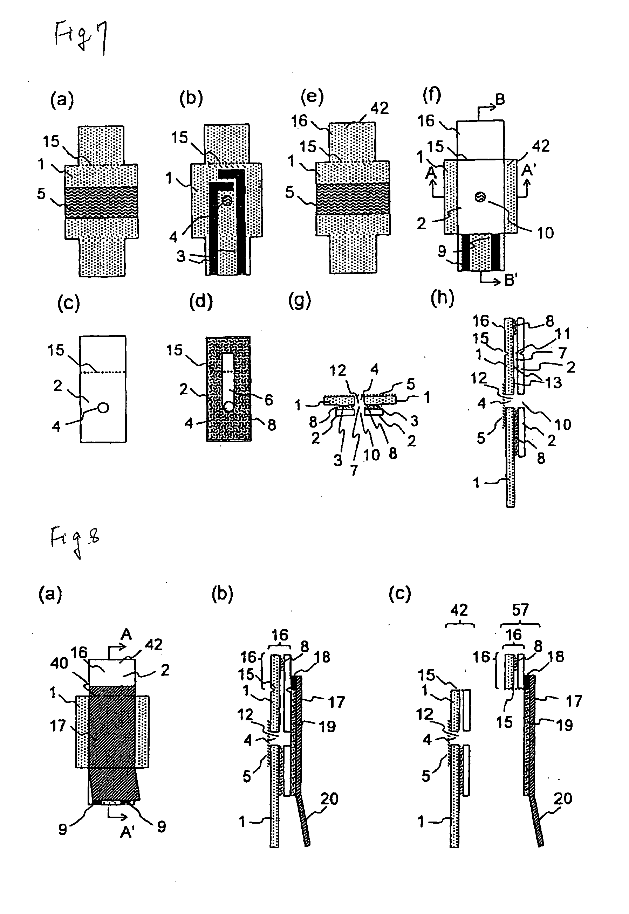

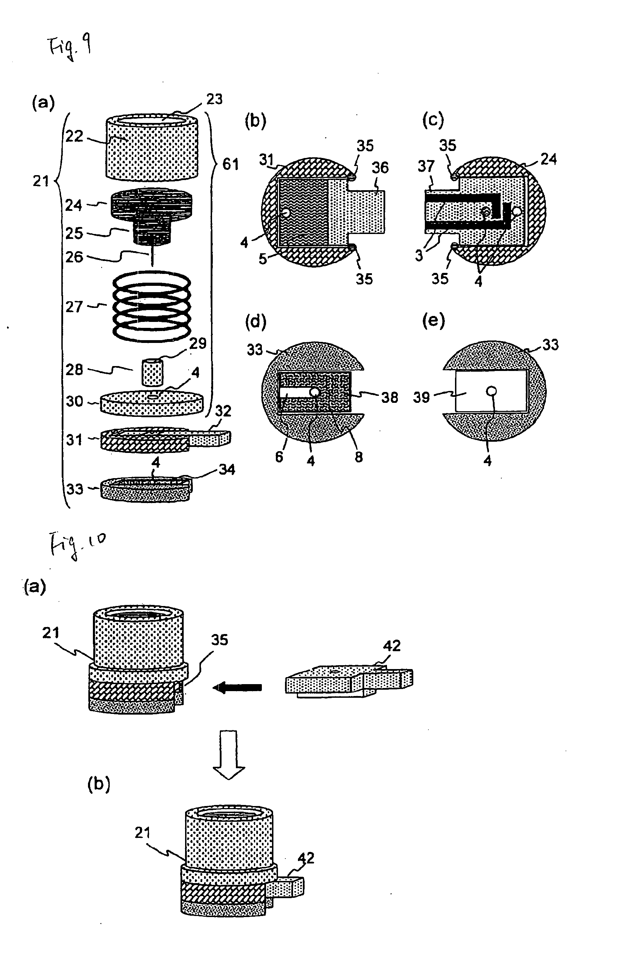

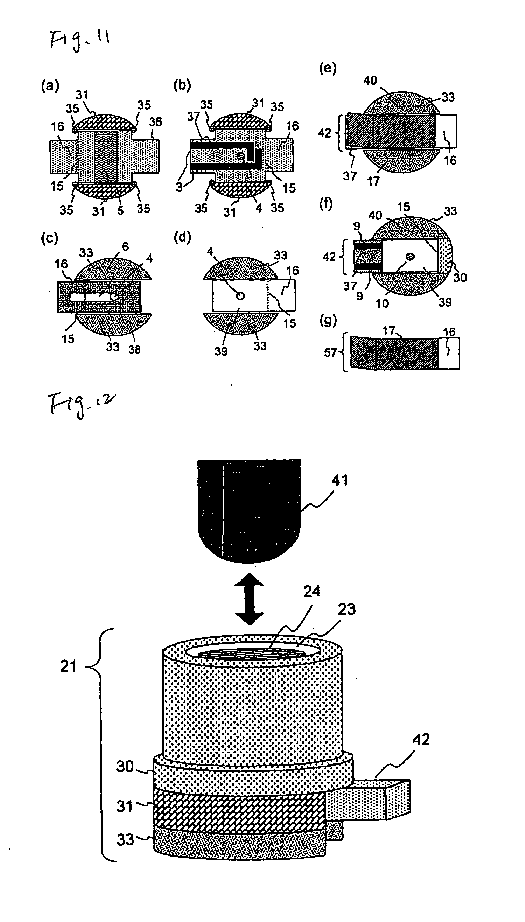

In Fig. 2, an improvement is done so that the collected blood is efficiently introduced and the blood fluid more than the necessary amount is made hardly collected thereinto in the biosensor shown in Fig. 1. Such a characteristic appears in the three parts as shown below.
(1) Diameter of a penetrating hole 4 of a cover part 2 is made larger than that of a substrate 1 (as compared (b) with (c)). As a result, the collected blood coming into an opening 10 for collecting the blood of the cover part 2 is apt to arrive the substrate part 1 due to its surface tension.
(2) In order to quickly move the collected blood flown from the opening 10 for puncturing and collecting the blood to an electrode reaction part 13, width of a conveying path 7 for the collected blood at the side of the electrode reaction part 13 is partly made big (refer to (d)). As a result, the blood collected at the opening for puncturing and collecting the blood is apt to come to the electrode reaction part 13 due to a taper structure where the conveying path 7 for the collected blood becomes narrow from the opening 10 for puncturing and collecting the blood to the electrode reaction part 13.
(3) As a result of making the width of an air outlet 11 narrow, a body fluid more than the necessary amount is apt be hardly taken therein (refer to (d)).

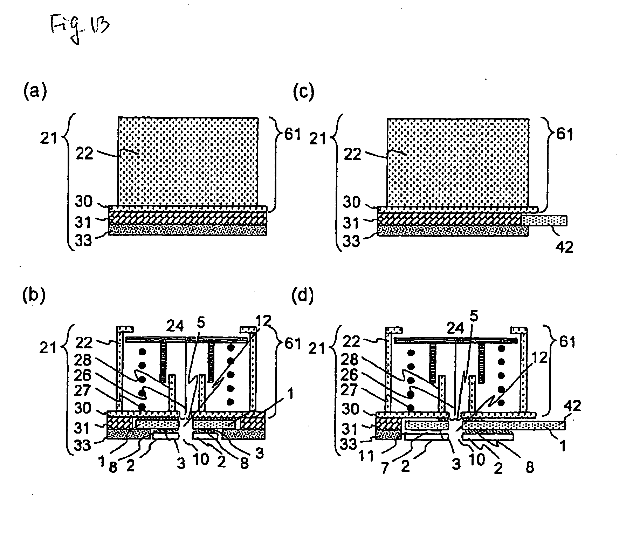

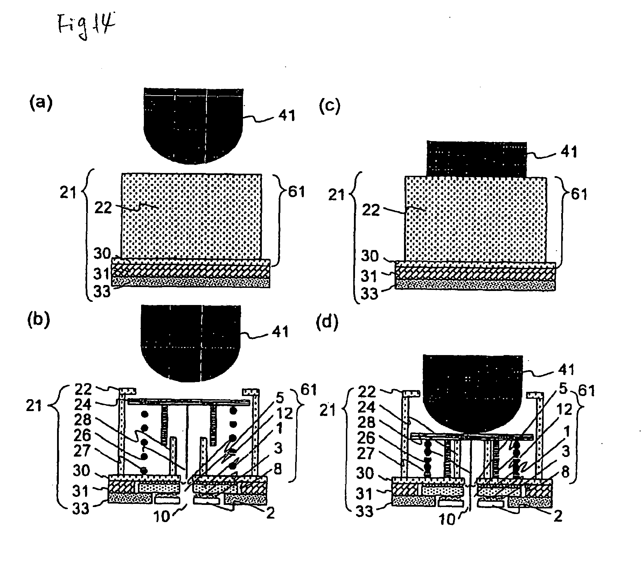

Fig. 3 is an example where an introducing guide 14 for the collected blood is attached to the outer block of a biosensor shown in Fig. 1 (refer to (c), (f), (g) and (h)). With regard to the introducing guide 14 for the collected blood, although there is no particular choice for its material, a material which is elastic and hardly slips is preferred if at all possible. As a result of attaching such a guide, the puncturing part is sensually grasped and, further, an anti-slipping effect works upon puncturing so that the skin of the person to be tested can be pushed thereto whereby it is possible that the area to be punctured is risen up and collecting the blood by puncturing is made easier.

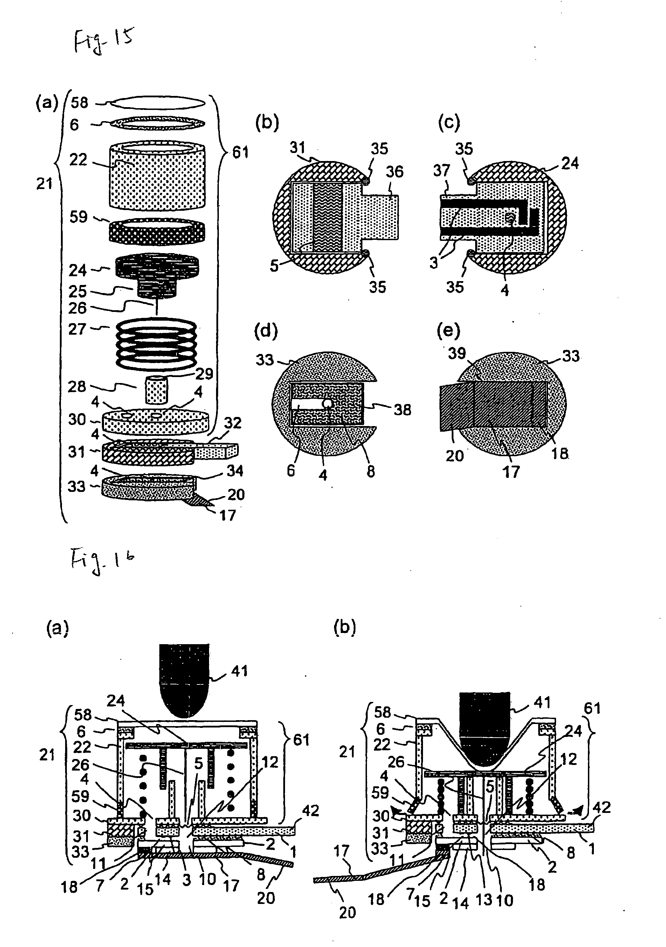

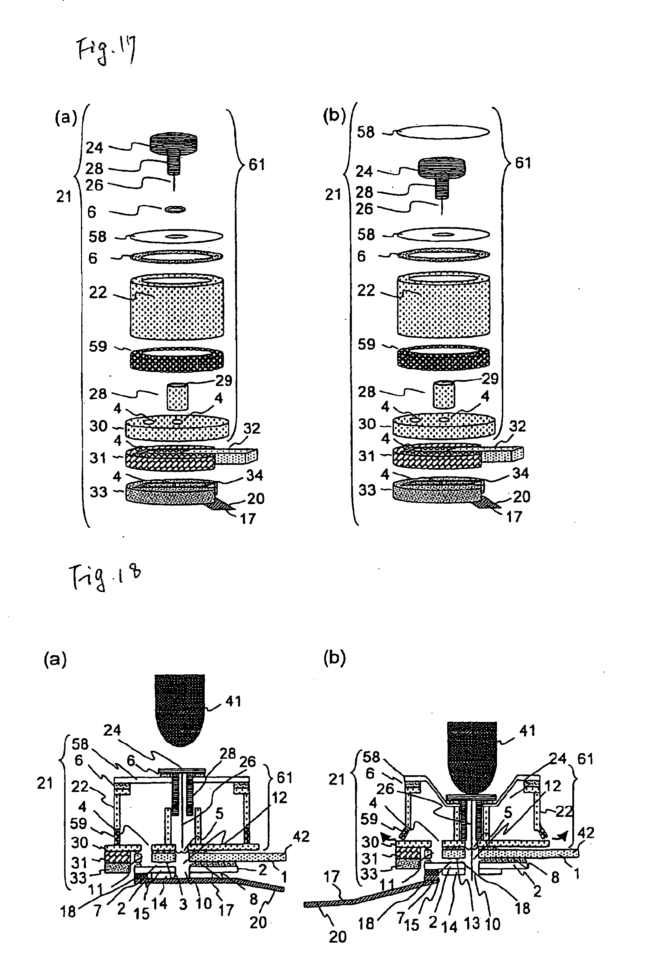

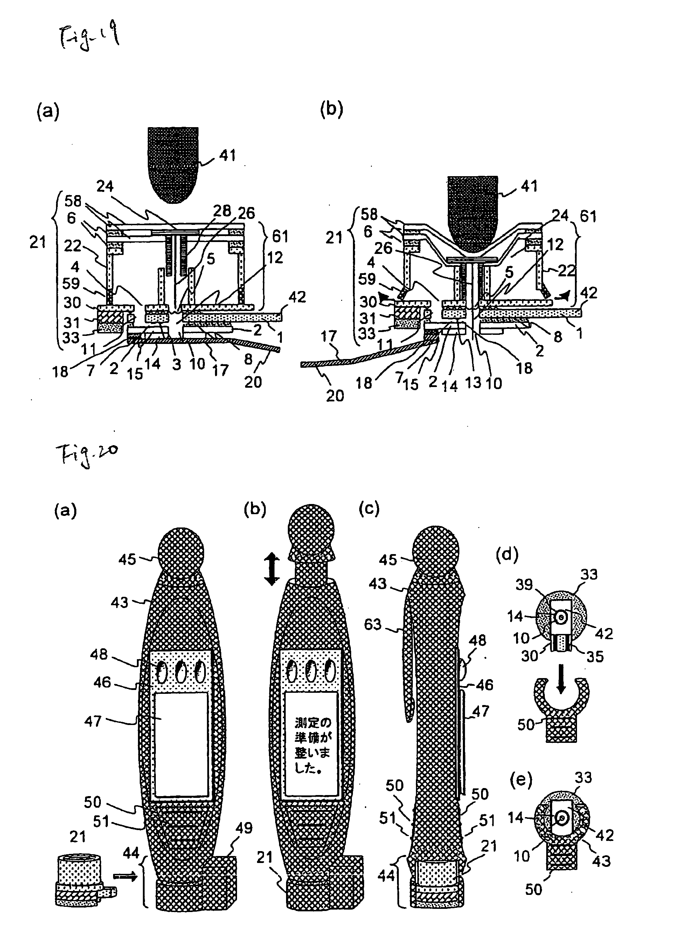

Fig. 4 is an example where an introducing guide 14 for collecting the blood is attached around an opening 10 for puncturing and collecting the blood of a biosensor shown in Fig. 1 (refer to (c), (f), (g) and (h)). Although there is no particular choice for the introducing guide 14 for collecting the blood, that in a gel form is preferred if at all possible. As a result of attaching the guide as such, the outcome is the same as in the biosensor shown in Fig. 3 that the puncturing part is sensually grasped and, upon puncturing, an anti-slipping effect and a close contact to the skin are able to be enhanced. As a result, it is possible to avoid the waste such as that the body fluid collected at the puncturing area is impregnated with a cover 2 material. Further, in the case of a biosensor as shown in this drawing, it is possible to enhance the close adhesion to the skin as mentioned above and, therefore, the collected blood is sucked utilizing a negative pressure generated in a housing 61 (not shown) for the puncturing needle and the collected blood can be quickly transferred to an electrode reaction part 13. In order to make the collecting of the blood by suction possible, the biosensor shown in this drawing adopts such a structure that a puncturing membrane 5 is sandwiched and closely attached between the surface of the substrate 1 and the housing 61 for the puncturing needle, a penetrating hole for an air outlet 11 is formed on the penetrating membrane 5 (refer to (a), (e), (g) and (h)) and the exhaust gas sucked by negative pressure is sent into the housing 61 for a puncturing needle. Accordingly, as to the puncturing membrane at that time, that which has a close adhesion to the substrate such as an artificial skin may be used.

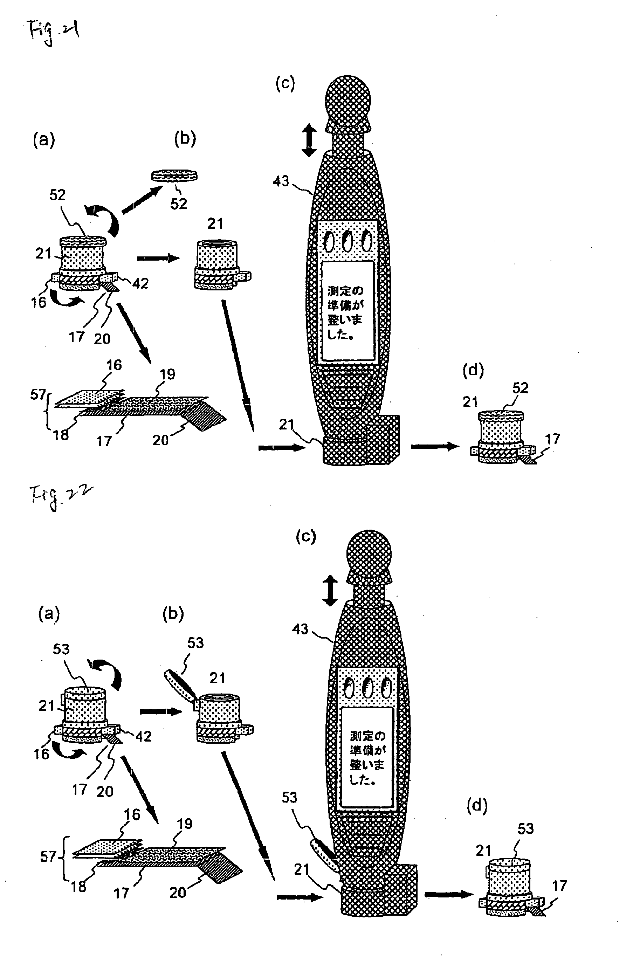

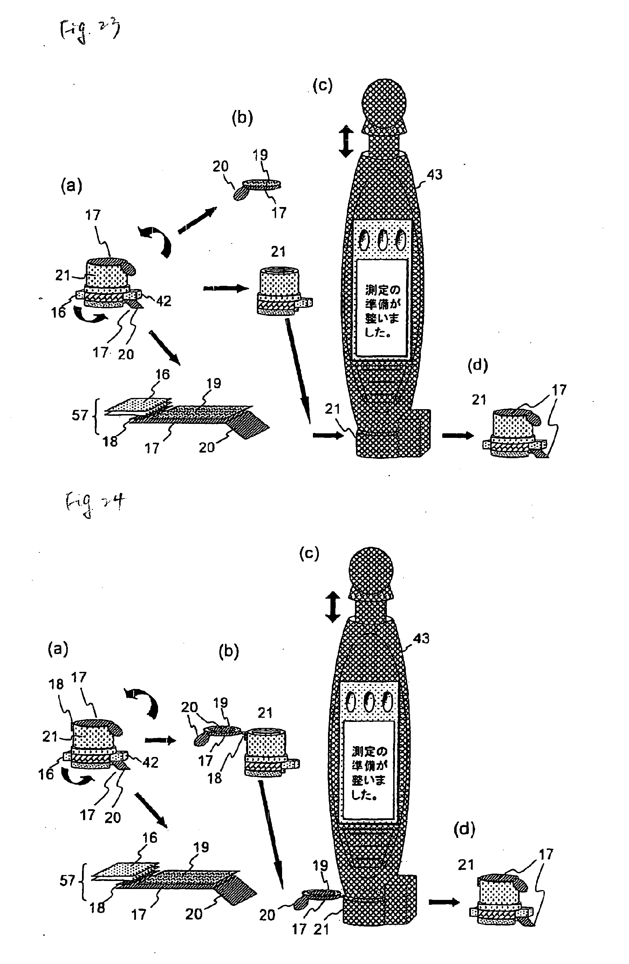

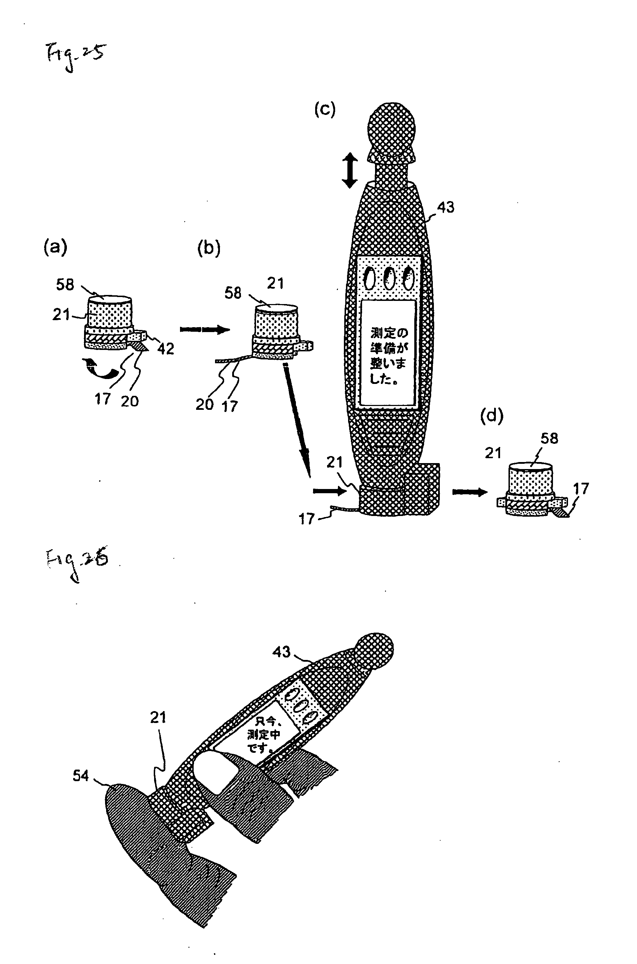

Fig. 5 is a simple packing example of the biosensor shown in Fig. 4. Fig. 5(a) shows a surface side of a cover 2 where a introducing guide 14 for the collected blood is formed around an opening 10 for puncturing and collecting the blood. Fig. 5 (b) shows a state where the area from the surface of the cover 2 of the biosensor shown in Fig. 4(f) to a terminal 9 on the substrate is covered by a protective film 17. Fig. 5 (c) is a cross-sectional view along A-A' shown in (b). The protective film 17 is fixed by a strongly adhesive layer 18 within a range from the upper part of the surface of the cover 2 to a folding 15 of the protective film 17. On other part of the protective film 17, an adhesive layer is not particularly formed (non-adhesive part 20). However, as a result of a simple adhesion to an introducing guide 14 for the collected blood in a gel form which is one of the characteristics of the biosensor shown in Fig. 4, the opening 10 for puncturing and collecting the blood is covered. Fig. 5 (d) shows an example how to use the biosensor which is simply packed by the protective film 17. When the protective film 17 is turned over until the folding 15, the biosensor is now ready for the measurement. Further, re-packing is possible by re-adhesion of the protective film 17 after use whereby the risk of infection can be avoided.

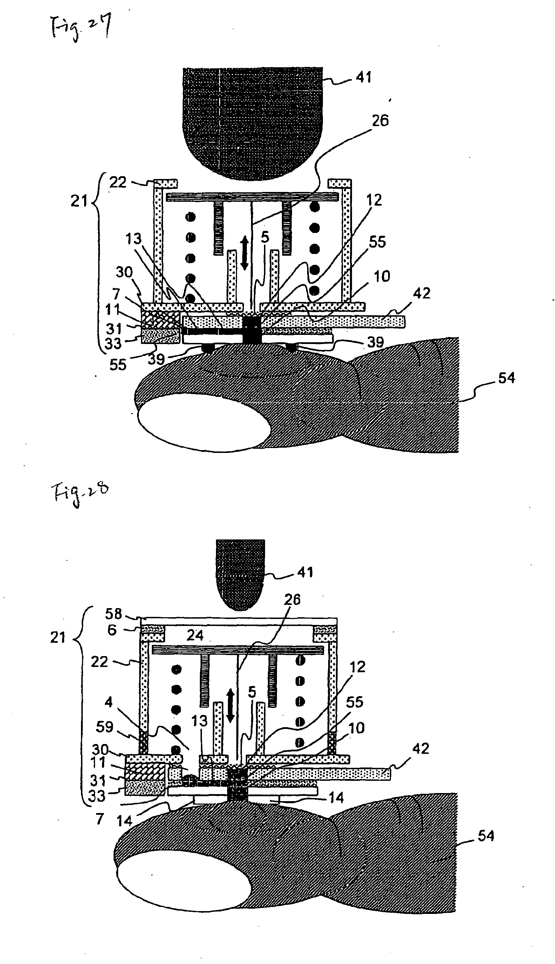

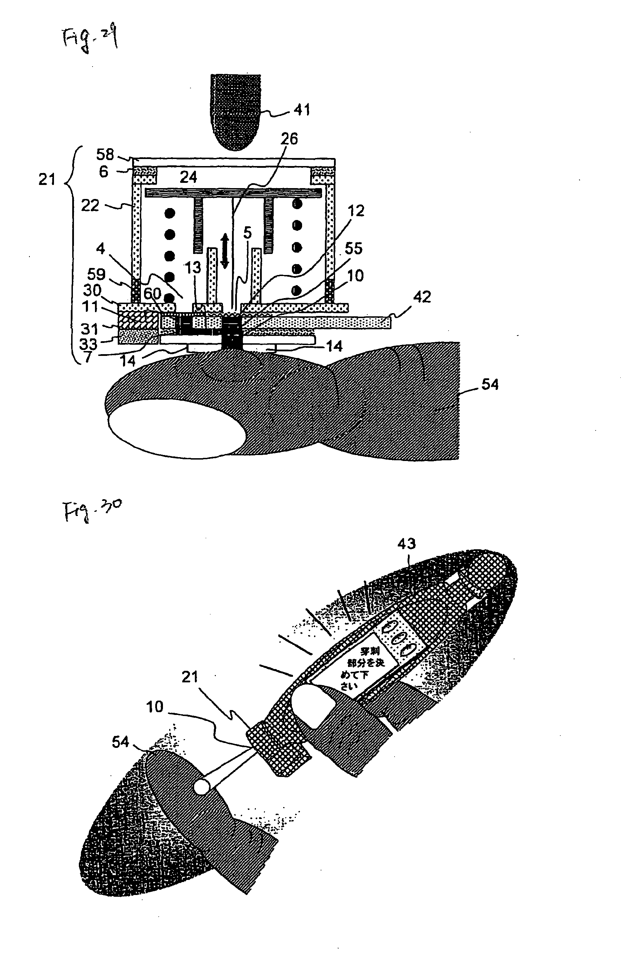

Fig. 6 shows an example where a gas-permeable film 60 is formed on the air outlet 11 of the biosensor shown in Fig. 4. On the back side of the substrate 1 of Fig. 6(a), a gas-permeable film 60 is adhered besides a puncturing membrane 5. The gas-permeable film 60 covers a penetrating hole 4 shown in (b). As will be noted from Fig. 6(h), this penetrating hole 4 becomes an air outlet 11. The characteristic of the biosensor shown in this drawing is a construction that, like in the biosensor shown in Fig. 4, the collected blood is sucked by a negative pressure taking place after puncturing at the housing 61 for a puncturing needle and is sent into the inner part of the biosensor and that flowing of body fluid more than the necessary amount caused by an excessive suction which may happen at that time is limited until the gas-permeable film formed at the air outlet 11.

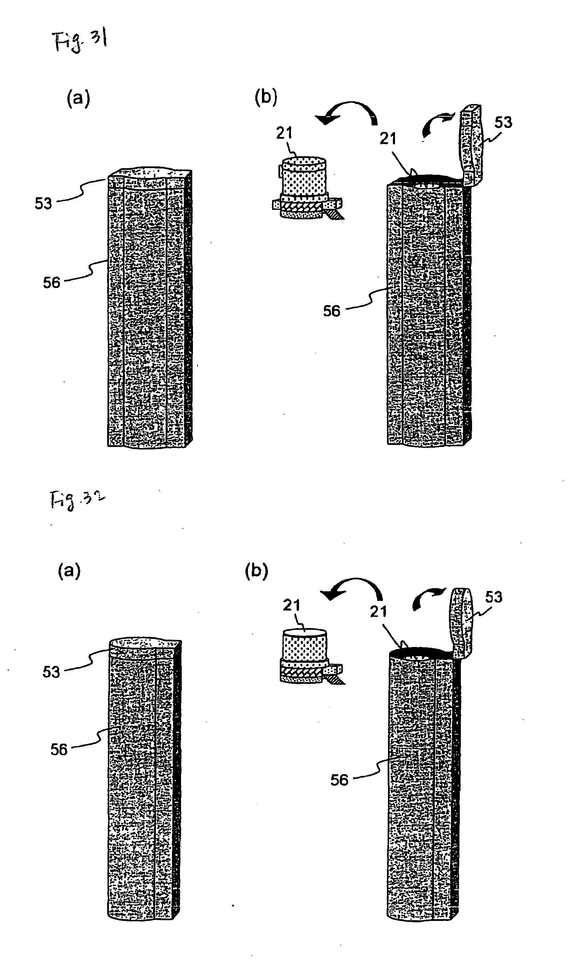

Fig. 7 and Fig. 8 show examples where the structure of the most basic biosensor of the present invention shown in Fig. 1 is improved. In the biosensor, an attempt is done so as to keep the inner part of a biosensor in a tightly closed state until use and to stably keep the state of the reagent inside for a long period. In Fig. 7(a), a plate material is extended upward on the substrate 1 shown in Fig. 1 (a) where a groove 15 is a boundary. In Fig. 7(b), a groove 15 on a substrate 1 is formed without crossing the electrically conductive materials 3. A cover 2 shown in Fig. 7(c) is also similar and a plate material is extended upward on the cover 2 shown in Fig. 1 (c) where the groove 15 is a boundary. In Fig. 7(d), the groove 15 on the cover 2 is formed crossing the non-spacer part 6. Figs. 7(e) and (f) show the substrate side 1 and the cover side 2, respectively, of a main body 42. Figs. 7(g) and (h) show cross sections along A-A' and B-B', respectively. As shown in Fig. 7(h), the groove 15 is formed to divide a conveying path 7 for the collected blood.

Fig. 8 shows an example of the state of a package of a biosensor shown in Fig. 7 with a protective film. Fig. 8 (a) shows an example where the outside of a cover part 2 of a biosensor 42 is packed with a protective film 17. Fig. 8 (b) shows a cross section along A-A' of (a). In this drawing, a detachable layer 19 is newly formed on the protective film 17 used. Here, the detachable layer 19 means a sticking layer having a weak adhesive force where adhesion and detachment can be repeatedly conducted unlike the adhesive agent used in a strong adhesive layer 18. Fig. 8(c) shows a use example of the biosensor simply packed by the protective film 17 based on the cross section along A-A' of Fig. 8 (b). When a biosensor is divided along the V-shaped groove formed on a substrate 1 and the cover 2 part, a tightly closed cap part 16 containing no electrode pattern is detached, an air outlet 11 newly opens and an opening 10 for puncturing and collecting the blood is also opened by detachment of the detachable layer 19 part of the protective film 17. At that time, the tightly closed cap part 16 is also detached from a main body 42 of the biosensor as a sensor detaching part 57 together with the protective film 17 fixed by a strong adhesive layer 18. After the main body 42 of the biosensor is used, this sensor detaching part 57 can be used for adhering again as in the original state and for repacking.

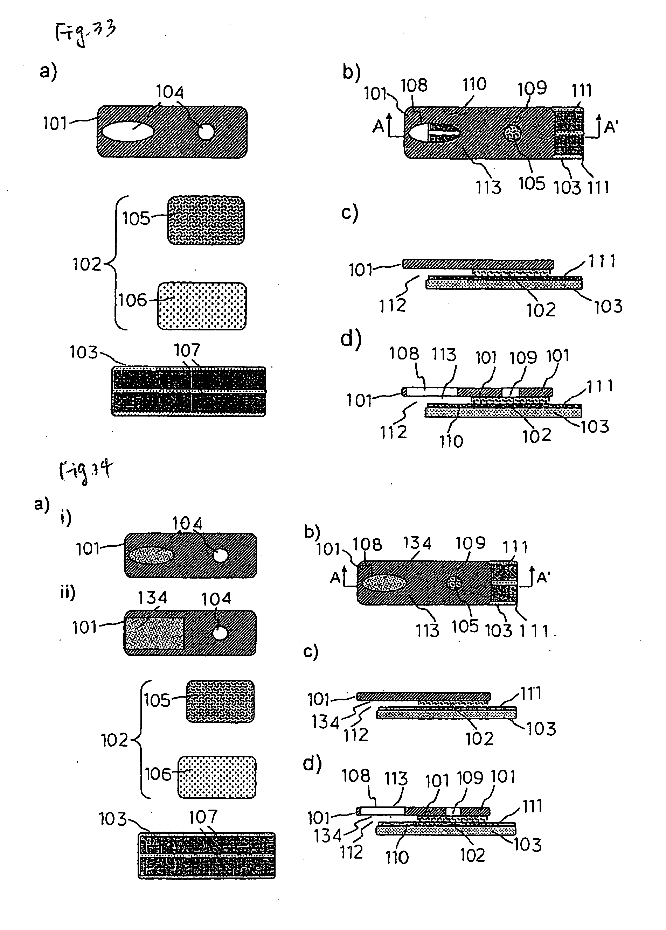

Fig. 9 shows an example of a constitutional drawing of a biosensor unified with a needle of a cartridge type. Incidentally, the biosensor which is shown here is the same one as shown in Fig. 1. Fig. 9 (a) shows a disassembled drawing of a biosensor 21 unified with a needle. The biosensor 21 unified with a needle is in such a constitution that the upper part is a puncturing needle while the lower part is a biosensor. A puncturing needle 26 is fixed to a substrate 24 for fixing the puncturing needle and, on the outer frame thereof, a coiled spring guide 25 for avoiding the contact to a coiled spring 27 and to stipulate the moving direction of the coiled spring is installed. On the lower part of the puncturing needle 26, there is a puncturing needle folder substrate 30 and, in this substrate, a penetrating hole 4 for the puncturing needle and a guide 28 for the puncturing needle for protection of the orbit of the puncturing needle from inner side are installed. In a space between a puncturing needle folder 22 and the puncturing needle folder substrate 30, parts necessary for puncturing are received forming a housing 61 for the puncturing needle.

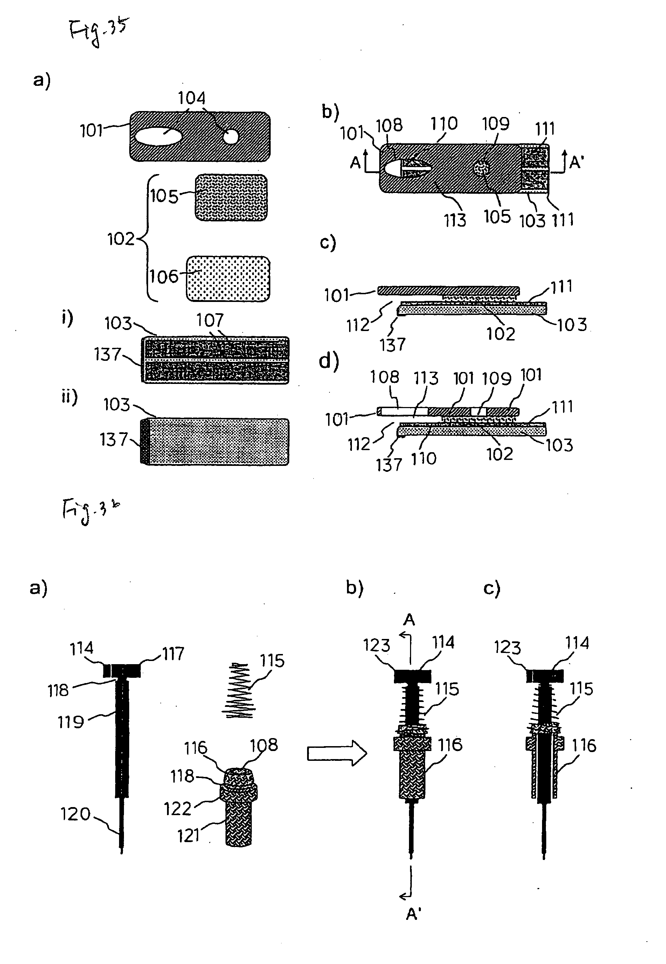

In the part beneath the housing 61 for the puncturing needle, two folders are installed for preventing the detachment of the biosensor 42 by the shock upon puncturing. Figs. 9(b) and (c) show the cases of the states where the sensor substrate 1 (32) is inserted into a sensor substrate folder 31 is observed from the upper area and the lower part, respectively. Figs. 9(d) and (e) show the cases of the states where the sensor cover 2 (34) is inserted into a sensor substrate folder 33 are observed from the upper area and the lower part, respectively. As shown in Figs. 9 (b) to (e), those folders are in such a structure where the sensor cover 34 and sensor substrate 34 having different shapes are inserted following the shapes in order to prevent the detachment of the biosensor due to puncturing. The sensor cover folder 33 is installed under the sensor substrate folder 31 for fixing the sensor substrate 32 having a bigger size than the cover 34 and, as a result, the substrate part poking out of the cover 34 is supported by the sensor cover folder 33. Such a support is helpful for preventing the detachment of the biosensor 42 due to the shock upon puncturing and, moreover, in assembling the biosensor unified with a needle, it is convenient for inserting the biosensor 42 by sliding on those folders. Further, as shown in Figs. 9(b) and (c), a fixing means 35 for sensor substrate is installed in a sensor substrate folder 31 so that the sensor substrate 32 is inserted into the substrate folder 31 by sliding and is surely fixed causing no detachment. Figs. 10 (a) to (d) show examples of assembling the biosensor 21 unified with a needle shown in Fig. 9.

Fig. 11 shows the case where the biosensor 42 shown in Fig. 7 and Fig. 8 is assembled as a biosensor unified with a needle. Figs. 11 (a) and (b) show the cases in which the state where a sensor substrate 32 is inserted into a sensor substrate folder 31 are observed from the upper area and the lower part, respectively. Figs. 9 (c) and (d) show the cases in which the state where a sensor cover 34 is inserted into a sensor cover folder 33 are observed from the upper area and the lower part, respectively. As shown in Figs. 11 (a) and (b), in order that the biosensor of this form is received in and fixed to a cartridge for a biosensor unified with a needle, it is necessary that four places are fixed with a fixing means 35 for the sensor substrate 32. Fig. 11(e) shows that case where the state in which the biosensor 42 in a packed state is received in a cartridge is observed from the lower area. Here, a broken line 40 shows an outer frame part of the sensor substrate 32 when seeing therethrough. It is noted from the broken line 40 as well that the biosensor is received in a folder in a slid form and is well durable against the shock upon puncturing. Fig. 11(f) shows the case when the packing for the biosensor is unpacked from the state of (e) and a sensor detached part 57 which is detached at that time is shown in Fig. 11 (g). It is noted that, in such an embodiment, repacking of a biosensor after use is easy by re-adhesion.

Fig. 12 is a drawing in which the position relationship of a biosensor 21 unified with needle and an external driving 41 is shown in a three-dimensional manner. As shown by this drawing, there are a structure where the driving from outside comes into an introducing part 23 for driving from outside opening at the head of a puncturing needle folder 22 and a structure where the external driving 41 is transmitted to the upper part of the fixing substrate 24 for the puncturing needle in a puncturing needle housing 61.

Fig. 13 shows the case where the biosensor unified with a needle 21 shown in Fig. 9 is observed from the side area. Fig. 13(a) shows the state where the main body of the biosensor of the biosensor unified with a needle 21 turns its head to the front and Fig. 13(b) shows a cross section thereof at that time. Fig. 13(c) shows the state where the main body of the biosensor of the biosensor unified is observed from the side area and Fig. 13 (d) shows a cross section thereof at that time.

Fig. 14 shows the biosensor unified with a needle 21 shown in Fig. 13 together with the behavior of the puncturing driving. From the cross sections, it is noted that, in Fig. 14(b), a puncturing needle 26 is located at the center in the housing 61 for the puncturing needle and, below that, a penetrating path for a puncturing needle is installed across the penetrating membrane 5. Fig. 14(d) shows the state where, upon a puncturing driving, the front end of the puncturing needle moves downwardly, penetrates through the biosensor and projects the outer part of the cartridge for short time.

Fig. 15 shows an example where a stretchable film is applied to a biosensor 21 unified with a needle. Difference in terms of construction between Fig. 15(a) and the biosensor unified with a needle shown in Fig. 9(a) is that a stretchable film 58 is fixed by an adhesive on the upper part of the housing 61 for a puncturing needle, a valve for preventing the back-flow in order to adjust the inner gas pressure is installed in the housing 61 for a puncturing needle, a penetrating hole for suction of the collected blood is formed on the substrate 30 for the puncturing needle folder and, as shown in Figs. 15(b) to (e), packing is possible in a biosensor and suction of the collected blood by negative pressure resulted in the puncturing needle housing is possible, etc.

Fig. 16 shows the behavior of an inner area by the driving from outside in a cartridge of a tightly closed type shown in Fig. 15. Fig. 16(a) shows an inner area of the cartridge of a tightly closed type before use. Fig. 16 (b) shows the scene where a package 18 of the biosensor is unpacked and the puncturing driving works. As shown by this drawing, the puncturing needle 26 permeates the puncturing membrane 5 and the main body 42 of a biosensor and projects outside. At that time, as result of a sudden rise in the pressure, discharge of the inner air by a valve for preventing the backflow is conducted in the inner area of the housing 61 for a puncturing needle.

Fig. 18 shows an example of the case where a cartridge of a tightly closed type using a stretchable film shown in Fig. 15 (a) is used is improved. A coiled spring is used as an elastic matter in Fig. 15(a) while, in Fig. 17, supplement is done by the use of only a stretchable film 58 instead of the use of the coiled spring. Thus, in addition to the upper part of the housing 61 for a puncturing needle, a stretchable film 58 can be fixed to a substrate 30 for fixing a puncturing needle as well whereby no coiled spring is necessary. That is because, when the puncturing needle projects outside by a puncturing driving, the stretchable film 58 becomes to the most stretched state and the backlash thereof works as a driving force for returning the puncturing needle to the original state. Fig. 17 (a) shows the case where one sheet of a stretchable film 58 is fixed to a substrate 30 for fixing the puncturing needle and Fig. 17(b) shows the case where two stretchable films 58 are held and fixed on upper and lower parts, respectively, of the substrate 30 for fixing the puncturing needle.

Fig. 18 is a cross sectional view of a cartridge of a tightly closed type shown in Fig. 17 (a). Fig. 18 (a) shows the state before use while Fig. 18(b) shows the case where the package 17 of the biosensor is unpacked and a puncturing driving works.

Fig. 19 is a cross sectional view of a cartridge of a tightly closed type shown in Fig. 17(b) in which Fig. 19(a) shows the state before use while Fig. 19 (b) shows the case where the package 17 of the biosensor is unpacked and a puncturing driving works.

Fig. 20 shows an example of a measuring apparatus equipped with a puncturing driving for the measurement using a cartridge for a biosensor unified with a needle according to the present invention. Fig. 20(a) shows the state before the cartridge 21 is introduced into the measuring apparatus 43. The measuring apparatus will be illustrated by referring to this drawing. Said measuring apparatus 43 is constituted from a introducing part 44 of a cartridge 21 for a biosensor unified with a needle, a trigger part 45 for a puncturing driving, an operation panel 46, a connector 49 for biosensor terminal and an initiating button 50 for puncturing. A display 47 and an operation button 48 are installed on the operation panel 46 while, on the initiating button 50 for puncturing, an anti-slip device 51 is installed. Fig. 20(b) shows the state that a sensor cartridge 21 is introduced into a measuring apparatus 43, a trigger 45 on the upper part is pulled, the power source for the measuring apparatus turns on and the apparatus is now in a measuring mode. Letters displayed at that time are in an easily legible direction due to the operation of an automatic switching mode where they become in the same direction as the measuring apparatus is placed. Fig. 20 (c) shows the state after a sensor cartridge 21 is placed into a measuring apparatus 43 is observed from a transverse side. As the drawing shows, a hook 63 is formed on the back of the display whereby the main body of the measuring apparatus can be easily received in a breast pocket or an inside pocket. Fig. 20 (d) shows the state when the sensor cartridge 21 and the measuring apparatus 43 are observed from the lower side. In the biosensor 42 equipped to the sensor cartridge 21, an introducing guide 14 for the collected blood is attached to the contour of the opening 10 for puncturing and collecting the blood shown in Fig. 3. Further, on the bottom of the measuring apparatus or, in other words, on the bottom of the introducing part 44 for a sensor cartridge, an anti-slip device 51 not to cause discrepancy in the positions of the measuring apparatus and the skin upon puncturing is installed.

Fig. 21 shows a use example of a sensor cartridge 21 to which a simple package is applied. In Fig. 21(a), a detachable cap 52 covers the upper part of a housing 61 for a puncturing needle in the sensor cartridge 21 while a package by a protective film is applied to a biosensor 42. The sensor cartridge 21 is set with a biosensor of a tightly closed type shown in Fig. 7 and Fig. 8. Fig. 21(b) shows the state where a separating part 57 attached with a protective film of biosensor 42 and cap 52 are detached from a sensor cartridge 21 and before it is introduced into a measuring apparatus 43. Fig. 21(c) shows that the sensor cartridge 21 is set in the measuring apparatus 43, a switch for the display turns on and the measurement is ready. Fig. 21(d) shows the state where the sensor cartridge 21 after used for the measurement is taken out from the measuring apparatus 43, re-packed and is ready for discarding.

Fig. 22 shows another use example of a sensor cartridge 21 to which a simple package is applied as shown in Fig. 21. As shown in Fig. 22(a), on the upper part of the sensor cartridge 21, the same cap 53 as in Fig. 22 is attached to the upper part of the housing 61 for a puncturing needle and is connected by a cramp. Fig. 22 (b) shows the state the cap 53 is opened and before inserting it to a measuring apparatus. Fig. 22 (d) shows that the sensor cartridge 21 after used for the measurement is taken out from the measuring apparatus 43, re-packed and is ready for discarding. In that case, since the structure is that the cap 53 is not detached from the housing 61 for a puncturing needle, it no longer happens that the cap is lost after being used.

Fig. 23 and Fig. 24 show other use examples of the sensor cartridge 21 to which a simple package is applied using a protective film 17. The protective film 17 shown in Fig. 23 is a type which is detached from a cartridge while the protective film 17 shown in Fig. 24 is a type which is partly adhered to a cartridge strongly. Incidentally, the biosensor used 42 is the same as that shown in Fig. 22.

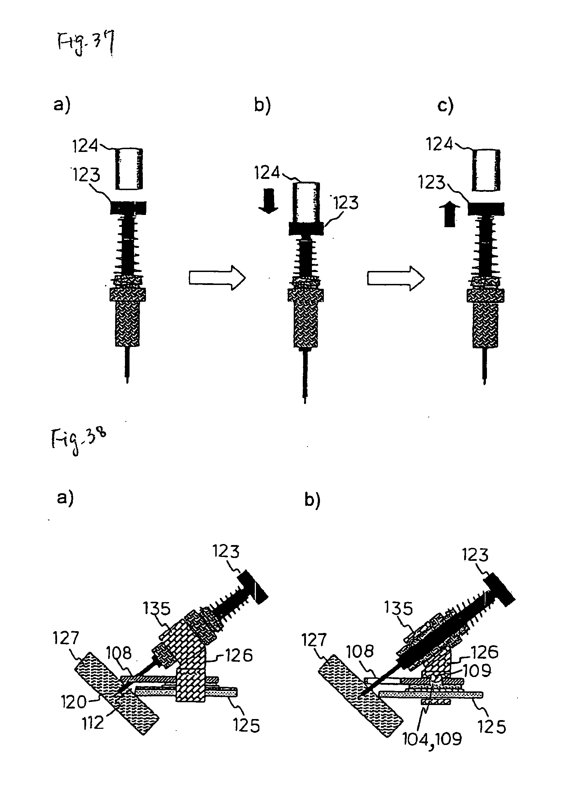

Fig. 25 shows a use example of a sensor cartridge 21 where a stretchable film 58 is applied to the upper part of the housing 61 for a puncturing needle. In the case of this sensor cartridge 21, it is possible to most simply start in the measurement only by an operation of detaching the protective film 17 of the biosensor 42 before use.

Fig. 26 shows the state where the biosensor unified with a needle according to the present invention is attached to a measuring apparatus, the measuring apparatus is attached to the finger which is a human body to be tested, an initiating button 50 for puncturing is pushed and the measuring mode after puncturing is resulted. As shown by this drawing, the screen of the measuring apparatus is displayed keeping its horizontal manner whereby the state in which the letters in the display are able to be easily recognized.

In Fig. 27, the situation in the sensor cartridge 21 during a measuring operation by the measuring apparatus shown in Fig. 26 is shown by way of a cross section along the longitudinal direction of the biosensor 42. The puncturing unit used here is that which is shown in Fig. 9 and, with regard to the biosensor 42, an air outlet 11 is installed at the extension of the conveying path 7 of the collected blood while an introducing guide 14 for the collected blood is installed at the contour of the opening 10 for puncturing and collecting the blood. This drawing shows the state where, when a finger 54 is pushed against the measuring apparatus 43, the skin which is a part to be punctured rises due to the introducing guide 14 for the collected blood. When the puncturing driving part 41 of the measuring apparatus is driven under such a state, a puncturing needle 25 is driven downward, penetrates the puncturing membrane 5 and the main body 42 of biosensor and projects to the finger. After that, the puncturing needle returns to the original position and, in the puncturing membrane 5, the permeating opening becomes small due to the character of its material and, at the same time, body fluid (blood) from the finger flows into the opening for puncturing and collecting the blood.

Fig. 28 shows a use example of the sensor cartridge 21 shown in Fig. 15 in terms of a cross section along the longitudinal direction of the biosensor 42. Said sensor cartridge 21 is equipped with a housing 61 for a puncturing needle covered with a stretchable film 58 and a biosensor where collecting the blood is possible by suction. The housing for a puncturing needle shown in the drawing is equipped with a valve 59 preventing the backflow and the biosensor 42 has a structure where it is possible to collect the blood by suction. Further, due to a gel-form introducing guide 14 for the collected blood attached to the opening 10 for puncturing and collecting the blood of the biosensor, there is a structure whereby a close contact to the skin is enhanced and collecting the blood in an efficient manner is possible.

Fig. 29 shows an example where, in a sensor cartridge shown in Fig. 28, a biosensor 42 equipped with a gas-permeable film 60 being formed at the air outlet 11 is installed. An object therefor is that the collected blood sent into a sensor by suction is retained in the gas-permeable film 60. As a result of the mechanism as such, incorporation of the collected blood in an amount of more than necessary can be avoided.

Fig. 30 shows an example of the measurement in a dark place. As the drawing shows, a backlight of the display automatically turns on in a dark place and the light illuminating a puncturing part is irradiated from the opening 10 for puncturing and collecting the blood.

Fig. 31 and Fig. 32 each shows the box 32 for receiving a sensor cartridge 21 in which a biosensor 42 having a different shape each other is installed. In the inner area of the box, it is possible that, for example, about ten sensor cartridges 21 are received and, when the cover 53 opens and closes, the sensor cartridge can be taken out.

Fig. 33 shows an example of the constitution drawing of a biosensor of the present invention. Fig. 33a) shows each of the constituting materials of the biosensor, and, from the top, a cover 101, an adhesive layer 105 and a resist layer 106 as a spacer 102 and a substrate 103 are shown. On the cover 101, two penetration holes 104 in ellipse and circle are formed along the center line in the longitudinal direction with an interval. Those two penetrating holes act as a penetrating path for a puncturing needle and as a position hole for fixing the biosensor to a puncturing needle part - biosensor folder, respectively. On the substrate 103, electrically conductive materials 7 acting as a terminal and an electrode are arranged. Fig. 33b) is a plane view of the biosensor where the members shown in Fig. 3a) are assembled. As the drawing shows, the cover is placed on the electrically conductive material 107 which is on the substrate 103. The electrically conductive material 107 exposed to the right end of the substrate 103 acts as a terminal 111. The adhesive layer 105 of the spacer 102 appears on the circular penetrating hole 104. The elliptic penetrating hole 104 acts as a penetrating path 108 for puncturing by which a puncturing needle passes through a cover part to arrive an opening for collecting the blood, and the surrounding thereof forms an opening 112 for puncturing and collecting the blood and an electrode reaction part 113. Fig. 33c) is an example of a side view of the biosensor shown in Fig. 33b). From the top, there are shown the cover 101, the adhesive layer and the resist layer as the spacer 102 and the substrate 103. On the left end of the biosensor, a space sandwiched between the substrate 103 and the cover 101 is formed, which forms the opening 112 for puncturing and collecting the blood. On the right end of the biosensor, a part of the substrate 103, on which the cover 101 is not arranged, forms the terminal 111. Fig. 33d) shows a cross section along A-A' in Fig. 33b). In the drawing, a stopper 109 is formed as a position hole for fixing the biosensor to the puncturing needle part - biosensor folder. Inn the penetrating path 108 for a puncturing needles, there is adopted a structure where a puncturing needle passes through the penetrating path 108, passes the opening 112 for puncturing and collecting the blood formed between the substrate 103 and the cover 101 and hits an object to be tested which is closely adhered to the opening 112 for puncturing and collecting the blood. In the above-mentioned structure of the biosensor, there is a characteristic that the puncturing needle is not polluted by a reagent, passes the penetrating path 108 for the puncturing needle and able to collect the blood from the object to be tested.

Fig. 34 is an example where a puncturing membrane 134 is formed in a penetrating path 108 for a puncturing needle of a biosensor shown in Fig. 33. Fig. 34a-i) shows the surface of a cover 101 while Fig. 34a-ii) shows the back thereof. The penetrating path 108 for a puncturing needle on the lower side of a cover 101 is covered by the puncturing membrane 134. Fig. 34b) shows a plane view of the biosensor where members shown in Fig. 34a) are assembled. As shown in the drawing, the puncturing membrane 134 is formed on the left lower side of the cover 101 so as to cover the penetrating path 108 for a puncturing needle and a terminal is formed on the right end. Fig. 34c) is an example of the side view of the biosensor shown in Fig. 34b) and the puncturing membrane 134 is formed from an opening 112 for puncturing and collecting the blood to the upper part of an electrode reaction part 113. Fig. 34d) shows a cross section along A-A' in Fig. 34b) and, the same as in Fig. 34c), the puncturing membrane 134 is formed from the opening 112 for puncturing and collecting the blood to the upper part of the electrode reaction part 113 so that the puncturing membrane 134 covers the penetrating path 108 for a puncturing needle. As a result of such a structure, the structure of the puncturing membrane 134 can be retained even after the puncturing, whereby there is achieved an effect that sending the collected blood by a capillary phenomenon to the electrode reaction part 113 can be conducted smoothly.

Fig. 35 is an example where an anti-slip device 137 is formed on the lower side of the left end of a substrate 103 of a biosensor shown in Fig. 33. Fig. 35a-i) shows a surface of the substrate 103 while Fig. 35a-ii) shows a back. The anti-slip device 137 is formed on the lower side of the left end of the substrate 103. Fig. 35a) is a plane view of the biosensor where the members shown in Fig. 35a) are assembled. Fig. 35c) shows its side view and Fig. 35d) shows its cross-sectional view. According to the structure as such, disagreement between the biosensor unified with a needles and the skin of the object to be tested upon puncturing hardly takes place, whereby an effect of safe and sure puncturing and collecting of the blood can be achieved.

Fig. 36 shows a constitutional drawing of a puncturing part. Fig. 36a) shows each constitutional material of the puncturing part and the puncturing part comprises a puncturing needle part 114, a coiled spring 115 and a penetrating path 108 for a puncturing needle. Here, the puncturing needle part 114 comprises an introducing part 117 for driving from outside, a fixing part 118 for coiled spring, a support 119 for a puncturing needle, and a puncturing needle 120. The penetrating path 108 for a puncturing needle comprises a folder 116 for puncturing needle, a fixing part 118 for coiled spring, an introducing part 121 for puncturing needle folder and a stopper 122 for a puncturing needle part folder. Fig. 36b) is an assembling drawing for a puncturing part 123 where the coiled spring 115 is arranged between the puncturing needle part 114 and the penetrating path 108 for a puncturing needle. In that case, a fixing part for a coiled spring is placed at the puncturing needle part 114 and the penetrating path 108 for a puncturing needle so that the coiled spring 115 can be surely fixed. Fig. 36c) is a drawing which shows a cross section along A-A' of the puncturing part 123, and the puncturing needle part 114 is in such a structure that it can slide in the penetrating path 108 for a puncturing needle.

Fig. 37 is a drawing which shows the action of a puncturing part 123. Fig. 37a) shows an example of arrangement of the puncturing part 123 and a driving from outside 124. Fig. 37b) shows a state where a puncturing needle part 114 slides in the inner area of a penetrating path 108 of a puncturing needle by the puncturing driving 124 from outside received on the upper part of the puncturing needle part 114. Fig. 37c) shows a state where a puncturing needle part returns to the original position by the reaction of a coiled spring after the puncturing.

Fig. 38 shows constitution and use example of a biosensor unified with a needles. Fig. 38a) shows an example of a side view of the case where the biosensor unified with a needle is pushed at a puncturing position of a body 127 to be tested and a puncturing operation is carried out. The biosensor unified with a needle is constituted from a biosensor 125, a puncturing part 123 and a puncturing needle part-biosensor folder 126 which fixes both of them. The biosensor unified with a needle has such a structure that, when the puncturing part 123 receives an driving from outside, a coiled spring shrinks, a puncturing needle 120 comes through a penetrating path 108 for a puncturing needle installed at a cover part 101 of the biosensor 125 and punctures an object to be tested which is located at the front end of an opening 112 for puncturing and collecting the blood. Fig. 38b) shows the embodiment of Fig. 38b) by way of a cross-sectional view and the puncturing part 123 is inserted in a sliding manner into a puncturing needle guide 135 of the puncturing needle part-biosensor folder 126 and is set there. Here, a stopper (a convex part) 109 of the puncturing needle part-biosensor folder 126 is inserted into a penetrating hole (concave part) 104 formed in the cover part 101, whereby a main body 125 of a biosensor is fixed in the folder 126.

Fig. 39 shows an action of a puncturing part shown in Fig. 37 as a biosensor unified with a needle and shows, by way of a front view, an example of puncturing to the object to be tested 127. Fig. 39a) shows a state where a main body 128 of a biosensor unified with a needle is arranged to a puncturing site on the finger of a object to be tested 127. Fig. 39b) shows a state where the driving from outside 124 works and the front end of the puncturing needle penetrates the body to be tested and Fig. 39c) shows the state where the object to be tested bleeds after puncturing and the blood 129 is sent to an electrode reaction part 113 from an opening 112 for puncturing and collecting the blood of the biosensor and is started in the reaction and the measurement.

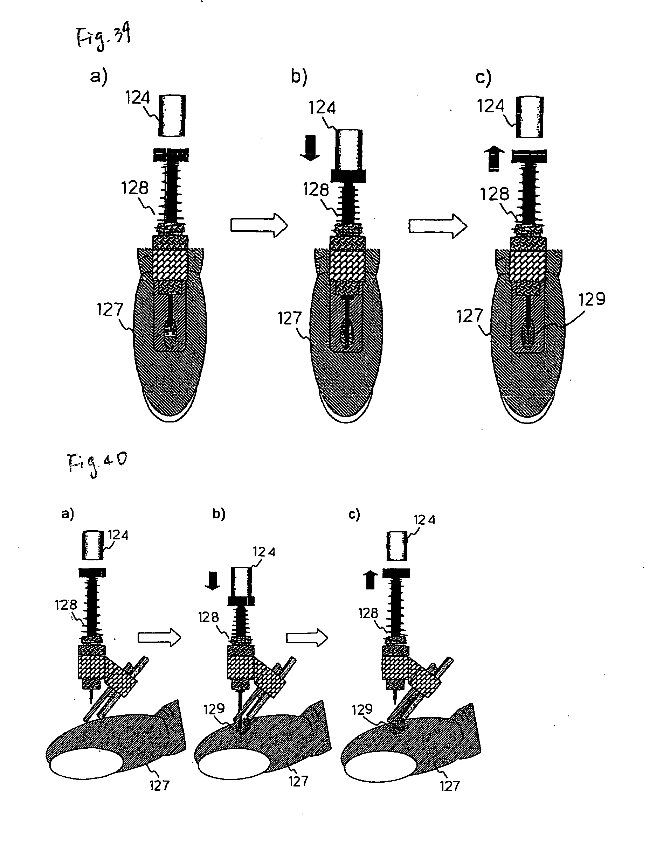

Fig. 40 shows an action of a puncturing part shown in Fig. 37 as a biosensor unified with a needle and shows an example of the puncturing into the object to be tested 127 by a side view.
Fig. 40a) shows the state where the main body 128 of a biosensor unified with a needle is arranged at a puncturing site on the finger of the object to be tested 127, Fig. 40b) shows the state where the driving from outside 124 works, the front end of a puncturing needle penetrates the object to be tested and bleeding starts and Fig. 40c) shows the state where bleeding takes place from the obj ect to be tested after puncturing and the blood 129 is sent to an electrode reaction part from an opening for puncturing and collecting the blood of the biosensor and is started in the reaction and the measurement. In that case, when a puncturing membrane shown in Fig. 34 is installed in a penetrating path for a puncturing needle of a biosensor, collecting the blood can be carried out more smoothly.

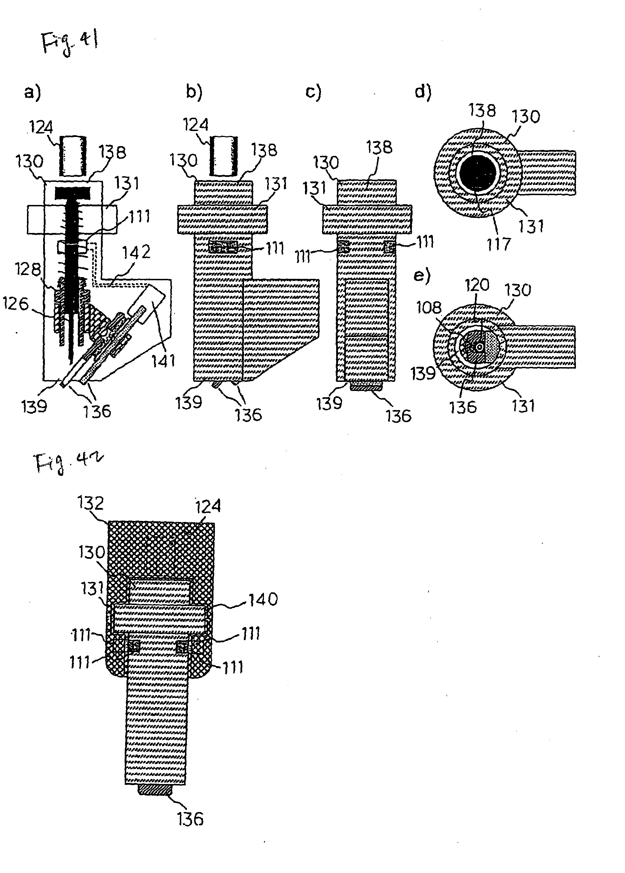

Fig. 41 shows the state where the biosensor unified with a needle according to the present invention is received in a housing 130. Fig. 41a) is a cross-sectional view showing an arrangement where the biosensor unified with a needle 128 is received in the housing 130. The housing 130 has an introducing guide 131 for a measuring apparatus, an upper opening 138 corresponding to an introducing opening for the puncturing driving 124, and a lower opening 139, and, as a result of fixation of the puncturing needle part-biosensor folder 126 and the housing 130, a biosensor unified with a needle 128 is enclosed. A terminal 111 of the biosensor is connected to a connector 141 installed in a housing 130 and is connected to a terminal 111 formed for connecting to outside of a housing 130 or, in other words, to a measuring apparatus by the wiring 142. In the lower opening 139, there is a housing projection 136 of a biosensor by which a puncturing place of an object to be tested can be sensually grasped by a user. In that case, it is also possible to install an anti-slip device to the projection 136. Fig. 41b) shows a side view of a housing 130. In the drawing, a terminal 111 is installed beneath the introducing guide 131 for a measuring apparatus for connecting to the measuring apparatus. Fig. 41c) shows a front view of the housing 130 and both terminals 111 are arranged in the same height. Fig. 41d) is a plane view of the housing 130. In the center of the upper opening 138, an upper part of an introducing part 117 of the driving from outside from a puncturing needle part receiving the external puncturing driving is arranged and an introducing guide 131 for a measuring apparatus surrounding the circumference of the upper opening 138. Fig. 41e) is an underside view of the housing 130. In the center of the lower opening 139, projection 136 of the front end of the biosensor is arranged, a penetrating path 108 for the puncturing needle is arranged in the central part thereof and, in the inner part thereof, a puncturing needle 120 is arranged so as to perpendicularly move to the object to be tested. As a result of such a structure, the puncturing needle 120 moves in the vertical direction of the housing 130 and can straightly penetrates the object to be tested by a puncturing driving 124 receiving in a perpendicular direction.

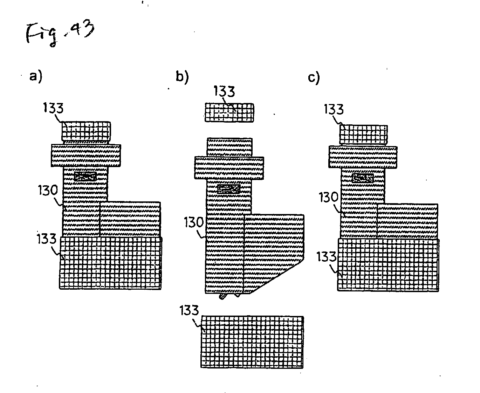

Fig. 42 shows a front view when a housing 130 for a biosensor unified with a needle is attached to a measuring apparatus 132 and the measuring apparatus 132 is equipped with a puncturing driving 124. The structure is in such a manner that a housing introducing part 140 is installed in the measuring apparatus 132 whereby the housing 130 is surely attached meeting the shape of the introducing guide 131 for a measuring apparatus and that the measuring apparatus 132 contacts the terminal 111 of the housing 130.

Fig. 43 shows an example of package of a housing 130 for a biosensor unified with a needle. Fig. 43a) shows the state where both upper opening 138 and lower opening 139 of the housing 130 are packed by a packing cap 133. As a result thereof, deterioration of the reagent arranged in the biosensor by humidity and direct sunlight can be prevented and accident by projection of a puncturing needle can also be avoided. Fig. 43c) shows the state where the packing cap 133 is detached and the measurement is ready. Fig. 43c) shows a packed housing for a biosensor unified with a needle which is re-packed after use and subjected to a treatment for avoiding the accident due to projection of a puncturing needle and the infectious disease resulted by the sample solution.

Although the present invention is illustrated in detail and by referring to specific embodiments hereinabove, it will be apparent to persons skilled in the art that various modifications and amendments thereof are able to be conducted without departing the spirit and the coverage of the present invention.
The present invention is based on the Japanese Patent Application (Patent Application No. 2005-057286) filed on March 2, 2005 and the Japanese Patent Application (Patent Application No. 2005-120478) filed on April 19, 2005 and their contents are incorporated herein as references.

## Claims

1. A biosensor unified with a needle where a puncturing needle for collecting a body fluid by puncturing a skin of a person to be tested and a main body of the biosensor for conducting an analysis of the body fluid are constituted in a unified manner and the puncturing needle is constituted in such a manner that it penetrates by driving the main body of the biosensor in a vertical direction by a driving means from outside and punctures the skin.

2. The biosensor unified with a needle according to claim 1,
wherein the biosensor is constituted from a spacer installed in a space sandwiched between an electrically insulating substrate and a cover, an electrode, a conveying path for a collected blood, an electrode reaction part, an opening for puncturing and collecting the blood, through which the puncturing needles penetrates to infuse the collected blood into the cover, and a substrate, which is equipped with a penetrating hole at a position opposite to the opening for puncturing and collecting the blood and a membrane (puncturing membrane) through which the needle can be penetrate, or a substrate equipped with a puncturing membrane only,
wherein the biosensor unified with a needle has a penetrating part for a puncturing needle where the puncturing needle can penetrate through the substrate and the cover,
wherein the biosensor unified with a needle has the electrode reaction part for an electrochemical measurement of the result of the reaction of a reagent in the electrode reaction part with a substance which is an object to be measured in the blood being resulted by such a manner that the blood flown out by puncturing of the skin of the person to be tested by the puncturing needle is introduced by capillary phenomenon from the opening for puncturing and collecting the blood to the conveying path for the collected blood and the electrode reaction part, and
wherein the biosensor unified with a needle is equipped with a folder for unifying the main body of the biosensor with the puncturing needle.

3. The biosensor according to claim 2, wherein the puncturing needle moving the penetrating part for the puncturing needle is arranged not to contact the reagent in the electrode reaction part.

4. The biosensor unified with a needle according to claim 2, wherein an opening for introduction of the sample and a pattern at an air outlet in the spacer sandwiched between the substrate and the cover are made in tapers.

5. The biosensor unified with a needle according to claim 2, wherein the puncturing membrane has a multi-layered structure comprising two or more layers having different materials.

6. The biosensor unified with a needle according to claim 2, wherein the puncturing membrane is a thin membrane having a thickness of not more than 100 µm.

7. The biosensor unified with a needle according to claim 2, wherein a shutter which opens and closes linking with the movement of puncturing needle is installed in the substrate of the biosensor.

8. The biosensor unified with a needle according to claim 2, wherein difference in level is made on the surrounding or the contour of the opening for puncturing and collecting the blood on a cover plane and the difference in level is used as an introducing guide for a sample.

9. The biosensor unified with a needle according to claim 2, wherein light of a visible light region is irradiated before puncturing from the opening for puncturing and collecting the blood to a puncturing position.

10. The biosensor unified with a needle according to claim 2, wherein a terminal on the insulating substrate is covered by the cover and the cover has a foldable folding line so as to expose an external terminal.

11. The biosensor unified with a needle according to claim 2, wherein a protective film, which can be adhered and detached, is placed on an outer surface of the insulating substrate.

12. The biosensor unified with a needle according to claim 2, further comprising a cap part for opening, the cap part having a cutting line on the boundary between a part of the insulating substrate containing no electrode and a part of the insulting substrate containing an electrode,
wherein an air outlet is opened when the part of the insulating substrate containing no electrode is cut off from the boundary.

13. The biosensor unified with a needle according to claim 2, wherein a package comprising a cap and a protective film can be re-adhered in the original state after using the biosensor.

14. The biosensor unified with a needle according to claim 1 or 2, wherein the puncturing needle is received in a housing for driving in a vertical direction and, the housing for the puncturing needle is equipped with such a mechanism that, after the puncturing needle driven by a driving means from outside penetrates the biosensor and punctures the skin of the person to be tested, it returns to the original position.

15. The biosensor unified with a needle according to claim 1 or 2, wherein a guide by which the action of the puncturing needle is made the same as the direction of the driving from outside is installed in a housing for the puncturing needle.

16. The biosensor unified with a needle according to claim 1 or 2, further comprising:
a mechanism for adjusting an inner pressure by which the pressure suddenly applied by the driving from outside in a housing for the puncturing needle in a tightly closed state is mitigated.

17. The biosensor unified with a needle according to claim 16, wherein the mechanism for mitigating the pressure is a valve for preventing a backflow.

18. The biosensor unified with a needle according to claim 1 or 2, wherein the inside of a housing for the puncturing needle which is in a tightly closed state after puncturing by the driving from outside becomes negative pressure and the negative pressure acts as a sucking force for introducing the collected blood to the conveying path.

19. The biosensor unified with a needle according to claim 1 or 2, wherein an air outlet necessary for introduction of the collected blood into the biosensor is equipped with a gas-permeable film.

20. The biosensor unified with a needle according to claim 1 or 2, wherein the puncturing needle has a photocatalytic action.

21. The biosensor unified with a needle according to claim 1 or 2, wherein an upper part of a housing for the puncturing needle is equipped with a cap for a simple package.

22. The biosensor unified with a needle according to claim 21, wherein the cap for the simple package is connected to the housing for the puncturing needle by a cramp.

23. The biosensor unified with a needle according to claim 1 or 2, wherein it is a cartridge type.

24. A cartridge for a biosensor unified with a needle according to claim 23, wherein it is a simple package type which can be re-packed.

25. A package for the cartridge of biosensor unified with a needle where plural cartridges for a biosensor unified with a needle mentioned in claim 23 or 24 are received.

26. A measuring apparatus for a biosensor unified with a needle, comprising:
the cartridge for the biosensor unified with a needle mentioned in claim 23 or 24;
an introducing part for the cartridge for the biosensor unified with a needles which slides and sets the cartridge for the biosensor unified with a needle;
a connector part which catches an electric signal at an electrode of the cartridge of the biosensor unified with a needle;
a measuring part which measures an electric value via the connector part;
an operation panel part for the measurement;
a display which displays the measured value at the measuring part;
a memory part which stores the measured value;
a driving part for the puncturing needle;
a trigger part for the driving part; and
a button for initiating a puncturing which initiates the puncturing by the puncturing needle.

27. The measuring apparatus for a biosensor unified with a needle according to claim 26, wherein a measuring method at the measuring part is a potential step chronoamperometry, a coulometry or a cyclic voltammetry.

28. The measuring apparatus for a biosensor unified with a needle according to claim 26 or 27, wherein the driving means from outside for the puncturing needle includes a mechanism such that, after it drives the puncturing needle, it returns the puncturing needle to an original position.

29. The measuring apparatus for a biosensor unified with a needle according to any of claims 26 to 28, further comprising:
a mechanism in which a depth of puncturing the skin of the person to be tested can be adjusted.

30. The measuring apparatus for a biosensor unified with a needle according to any of claims 26 to 29, wherein an illuminating mechanism works linking with pulling of a trigger of a puncturing driving of the measuring apparatus and the light irradiated from an introducing part for the collected blood illuminates a puncturing part of the person to be tested.

31. The measuring apparatus for a biosensor unified with a needle according to any of claims 26 to 30, wherein a measurement of hematocrit value is conducted by measuring a moving speed in the biosensor.

32. A method of using the measuring apparatus for the biosensor unified with a needle according to any of claims 26 to 31, wherein the cartridge for the biosensor unified with a needle is set in the measuring apparatus equipped with the driving means to the puncturing needle, a state where measurement is possible is resulted when pulling a trigger, a series of operations of puncturing, collecting the blood and measurement is automatically conducted when the front end of the measuring apparatus is touched to the person to be tested and the initiation switch for puncturing is pushed, and a result of the operations is displayed on the display of the measuring apparatus.

33. A biosensor unified with a needle where a biosensor where a biosensor, which includes an electrically insulating substrate and a cover, and a spacer, an electrode and a reagent layer, which are installed in a space sandwiched between the substrate and the cover, and a puncturing needle, which penetrates a skin of a person to be tested and collects a body fluid, are unified, wherein a cover part forming an opening for collecting the blood installed at a front end of a longitudinal direction of the biosensor has one penetrating hole for the puncturing needle.

34. The biosensor unified with a needle according to claim 33, wherein in the penetrating hole, a shutter, which opens and closes linking with a movement of a membrane through which the needle is penetrable (puncturing membrane) or a movement of the puncturing needle, is provided.

35. The biosensor unified with a needle according to claim 33, wherein an anti-slip device is installed at a lower surface of an end portion of the substrate forming an opening for collecting the blood.

36. The biosensor unified with a needle according to claim 33, wherein, when the puncturing needle passes the penetrating hole formed in the cover part of the biosensor, it is arranged not to contact the substrate.

37. The biosensor unified with a needle according to claim 33, wherein, when the puncturing needle penetrates, the needle is arranged at the angle of 20 to 90° to the biosensor.

38. The biosensor unified with a needle according to claim 33, wherein the puncturing needle is fixed by a folder for the puncturing needle.

39. The biosensor unified with a needle according to any of claims 36 to 38, wherein the biosensor is fixed by a puncturing needle part-biosensor folder.

40. The biosensor unified with a needle according to claim 33, wherein the biosensor unfired with the needle is attached to a housing equipped with a cartridge for connecting to a measuring apparatus.

41. The biosensor unified with a needle according to claim 40, wherein the opening for collecting the blood at the front end of the biosensor unified with the needle projects from the housing.
